(19) Europäisches Patentamt
European Patent Office
Office européen des brevets

(11) EP 0 422 790 B1

(12) EUROPEAN PATENT SPECIFICATION

(45) Date of publication and mention of the grant of the patent: **13.03.1996 Bulletin 1996/11**

(51) Int Cl.[6]: **C12N 9/10**, C12N 15/54, C12P 35/00, C12P 37/00

(21) Application number: **90310448.7**

(22) Date of filing: **25.09.1990**

(54) **Recombinant DNA expression vectors and DNA compounds that encode acyltransferase activity of aspergillus**

Rekombinante DNA-Expressionsvektoren und DNA-Komponenten, die für eine Acyltransferase-Aktivität von Aspergillus kodieren

Vecteurs d'expression d'ADN recombinants et composés d'ADN codant une activité acyltransférase d'aspergillus

(84) Designated Contracting States:
**AT BE CH DE DK ES FR GB GR IT LI NL SE**

(30) Priority: **27.09.1989 US 413401**

(43) Date of publication of application:
**17.04.1991 Bulletin 1991/16**

(73) Proprietor: **ELI LILLY AND COMPANY**
**Indianapolis, Indiana 46285 (US)**

(72) Inventors:
- **Miller, James Robert**
  **South Indianapolis, Indiana 46250 (US)**
- **Skatrud, Paul Luther**
  **Greenwood, Indiana 46143 (US)**
- **Tobin, Matthew Barry**
  **Indianapolis, Indiana 46227 (US)**

(74) Representative: **Hudson, Christopher Mark et al**
**Lilly Industries Limited**
**European Patent Operations**
**Erl Wood Manor**
**Windlesham Surrey GU20 6PH (GB)**

(56) References cited:
**EP-A- 0 336 446**

- **MOL. GEN. GENET., vol. 221, 1990, pages 332-330, Springer-Verlag; E. MONTENEGRO et al.: "Cloning, characterization of the acyl-CoA: 6-amino penicillanic acid acyl-transferase gene of Aspergillus nidulans and linkage to the iso-penicillin N synthase gene"**
- **FEBS LETTERS, vol. 262, no. 2, March 1990, pages 342-344, Federation of European Biochemical Societies; P.A. WHITEMAN et al.: "Acyl coenzyme A: 6- aminopenicillanic acid acyl-transferase from Penicillium chrysogenum and Aspergillus nidulans"**
- **GENE, vol. 83, 30th November 1989, pages 291-300, Elsevier Science Publishers B.V. (Biomedical Division); J.L. BARREDO et al.: "Cloning and characterization of the acyl-coenzyme A: 6-aminopenicillanic-acid-acyltransferase gene of Penicillium chrysogenum"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 422 790 B1

## Description

The sulfur-containing β-lactam antibiotics are the most important clinically, and the isolation of novel β-lactam compounds continues nearly six decades since the discovery of the penicillins by Fleming. The common structural feature of the penicillins and cephalosporins (including cephamycins) is the β-lactam ring.

These antibiotics are produced by a variety of prokaryotes and lower eukaryotes. The penicillins, exemplified by the compounds penicillin G (benzylpenicillin) or penicillin V, are produced by filamentous fungi, most notably Penicillium chrysogenum. The cephalosporins, first isolated as a product from the lower eukaryote, Cephalosporium acremonium (syn. Acremonium chrysogenum), are also metabolites of many prokaryotes, especially Streptomyces clavuligerus, S. lipmanii and S. cattleya that also produce cephamycins and other β-lactams such as oxypenams (clavulanic acid) and carbapenems (thienamycin).

The development of cell-free systems from β-lactam-producing organisms has allowed the establishment of the biosynthetic steps in the pathway of the sulfur-containing β-lactams (penicillins and cephalosporins).

The initial steps in the formation of penicillins in filamentous fungi (e.g., P. chrysogenum), and the cephalosporins produced by both prokaryotes and lower eukaryotes, are identical. ACV synthetase catalyzes the condensation of the amino acid precursors L-α-aminoadipate, L-cysteine, and L-valine to the tripeptide LLD-ACV. The next step forms the first β-lactam in the pathway by the cyclization of the tripeptide yielding isopenicillin N (IPN), the precursor to all penicillins, cephalosporins and cephamycins.

After synthesis of IPN, the pathways to cephalosporins and penicillins diverge. In Penicillium chrysogenum, for example, the α-aminoadipyl side chain of IPN can be exchanged for one of many (nearly 100 to date) hydrophobic side chains derived from the corresponding acyl CoA. One of the most familiar examples is the formation of penicillin G (benzylpenicillin) from phenylacetyl CoA and IPN. However, in the fungus C. acremonium, the α-aminoadipyl side chain is isomerized to produce penicillin N. The five-membered thiazolidine ring of the penicillin is then "expanded" to the six-membered dihydrothiazine ring that is characteristic of the cephalosporins. This reaction is catalyzed by deacetoxycephalosporin C synthetase (DAOCS) and produces the first cephalosporin in the pathway, deacetoxycephalosporin C (DAOC). Although the DAOCS activities from Cephalosporium acremonium and the gram-positive bacteria, Streptomyces clavuligerus and S. lactamdurans, have been extensively characterized, the remaining steps in the pathway are less well understood.

The present invention provides a gene encoding one or more activities involved in the production of penicillins, said gene being referred to as isopenicillin N:acyl CoA acyltransferase. The activity is also known as acyltransferase. The present invention expands the repertoire of beta-lactam biosynthetic enzymes which can be overproduced. This ability facilitates the bioconversion of substrate analogs to novel beta-lactams and strain improvement by increased gene dosage.

The present invention comprises DNA sequences that encode the bifunctional enzyme isopenicillin N:acyl CoA acyltransferase (acyltransferase). A gene encoding this enzyme is designated penDE (Ingolia, T.D. and Queener, S.W., Med. Chem. Rev. 9:245-264 (1989)). The bifunctionality is due to the enzyme's ability to catalyze the formation of penicillin G from phenylacetyl CoA and either 6-aminopenicillanic acid or isopenicillin N. The reaction is a critical step in the biosynthesis of the important antibiotic penicillin G. This pathway is depicted in Figure 1.

The DNA compounds of the present invention encode the acyltransferase enzyme. The DNA compound that encodes the acyltransferase activity was isolated from Aspergillus nidulans genomic DNA. The cloned acyltransferase gene is useful for increasing the yield of penicillin G in Aspergillus or Penicillium, particularly if the reaction catalyzed by acyltransferase is a rate-limiting step. This is accomplished by increasing the gene dosage and/or placement of the coding region behind a strong promoter. Another use of the cloned gene (in modified form) is to inactivate the Aspergillus nidulans acyltransferase gene through recombination or the use of anti-sense RNA, to produce a cephalosporin compound if the epimerase (cefD) and expandase/hydroxylase (cefEF) are introduced into the cell and expressed at the same time.

An analogous use can be accomplished with the Penicillium chrysogenum acyltransferase gene. The Aspergillus nidulans acyltransferase gene is used as a hybridization probe to isolate the Penicillium chrysogenum acyltransferase gene by methods familiar to those skilled in the art. Large yields of cephalosporins in Penicillium can be achieved by the inactivation of the acyltransferase gene and the addition of genes encoding the enzymes epimerase (racemase) and expandase. The acyltransferase gene inactivation can be accomplished by gene replacement with the cloned gene which, for example, has an antibiotic resistance gene inserted into it.

The DNA compound that encodes the acyltransferase activity was isolated from Aspergillus nidulans genomic DNA and can be used to construct recombinant DNA expression vectors. Three types of these expression vectors are especially useful: the first drives high-level expression of the acyltransferase protein in E. coli; the second in Penicillium; and the third in Aspergillus.

The following section provides a more detailed description of the present invention. For purposes of clarity and as an aid in the understanding of the invention, as disclosed and claimed herein, the following items are defined below.

Acyltransferase activity - an enzymatic activity that catalyzes the formation of penicillins from an acyl CoA and either 6-aminopenicillanic acid or isopenicillin N.

aIPNS - isopenicillin N synthetase or isopenicillin N synthetase-encoding DNA from Aspergillus nidulans.

amdS - an acetamidase gene; also used in the Figures to denote the Aspergillus nidulans acetamidase gene.

amdSp - the promoter of the amdS gene.

AmR - the apramycin resistance-conferring gene; also used to denote the apramycin-resistant phenotype.

Antibiotic - a substance produced by a microorganism that, either naturally or with limited modification, will inhibit the growth of or kill another prokaryotic or eukaryotic cell.

Antibiotic Biosynthetic Gene - a DNA segment that encodes an activity necessary for an enzymatic reaction in the process of converting primary metabolites into antibiotics or converting one antibiotic compound into a different antibiotic compound.

Antibiotic-Producing Organism - any organism, including, but not limited to, Aspergillus, Streptomyces, Bacillus, Flavobacterium, Monospora, Cephalosporium, Paecilomyces, Podospora, Penicillium, and Nocardia, that either produces an antibiotic or contains genes that, if expressed, would produce an antibiotic.

Antibiotic Resistance-Conferring Gene - a DNA segment that encodes an activity that confers resistance to an antibiotic.

ApR - the ampicillin resistance-conferring gene; also used to denote the ampicillin-resistant phenotype.

AT - an acyltransferase gene.

bp - a base pair of double-stranded DNA.

cI857 - a gene encoding a temperature sensitive repressor of the λpL promoter.

Cloning - the process of incorporating a segment of DNA into a recombinant DNA cloning vector.

Coding sequence - the sequence of DNA in a gene that encodes either the amino acid residue sequence of the protein expressed by the gene or, in the case of rRNA or tRNA genes, the RNA sequence of the rRNA or tRNA expressed by the gene.

Cosmid - a recombinant DNA cloning vector that can replicate in a host cell in the same manner as a plasmid but that can also utilize phage packaging mechanisms.

Gene - a segment of DNA that comprises a promoter, translational activating sequence, coding sequence, and 3′ regulatory sequences positioned to drive expression of the gene product, either a protein (and thus necessarily an mRNA), tRNA, or rRNA.

Genomic Library - a set of recombinant DNA cloning vectors into which segments of DNA, which substantially represent the entire genome of a particular organism, have been cloned.

Hybridization - the process of annealing two single-stranded RNA and/or DNA molecules to form a double-stranded molecule that may or may not be completely base-paired.

IPS or IPNS - isopenicillin N synthetase.

Isopenicillin N - has the structure depicted below:

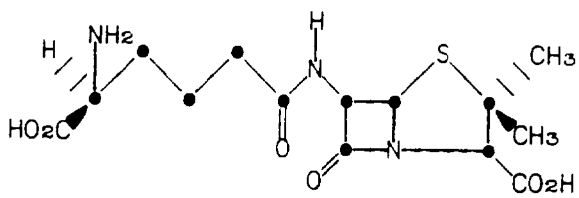

Isopenicillin N Synthetase - an enzyme, also known as cyclase, that catalyzes the formation of isopenicillin N from δ-(L-α-aminoadipyl)-L-cysteinyl-D-valine.

Isolated DNA compound - Any DNA sequence, however constructed or synthesized, which is locationally distinct from the genomic DNA of the organism in which the sequence occurs.

lacI - the E. coli lacI gene.

lacZα - the promoter and β-galactosidase (lacZ) α-fragment derived from the E. coli lac operon.

M13 ORI - the origin of replication of phage M13.

MCS - a multiple-cloning site.

mRNA - messenger ribonucleic acid.

ORI - a plasmid or vector origin of replication, the DNA sequence that serves as an attachment or start site for DNA polymerase.

pATp - the Penicillium chrysogenum acyltransferase promoter.

Pen DNA - DNA from Penicillium chrysogenum.

Penicillin G - has the structure depicted below:

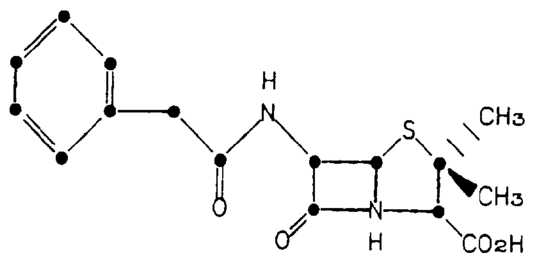

Penicillin N - has the structure depicted below:

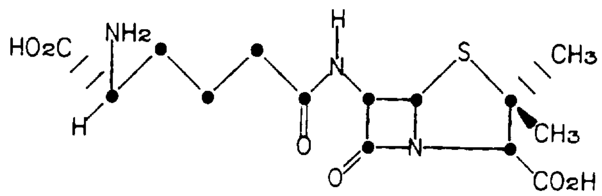

phlR - the phleomycin resistance gene.

pL - the leftward promoter from bacteriophage lambda.

Promoter - a DNA sequence that directs or initiates transcription.

Recombinant DNA Cloning Vector - any autonomously replicating or integrating agent, including, but not limited to, plasmids, comprising a DNA molecule to which one or more additional DNA molecules can be or have been added.

Recombinant DNA Expression Vector - any autonomously replicating or integrating agent, including, but not limited to, plasmids, comprising a promoter and other regulatory sequences positioned to drive expression of a DNA segment that encodes a polypeptide or RNA of research or commercial interest.

Recombinant DNA Vector - any recombinant DNA cloning or expression vector.

Restriction Fragment - any linear DNA molecule generated by the action of one or more enzymes.

rRNA - ribosomal ribonucleic acid.

Sensitive Host Cell - a host cell that cannot grow or whose growth is inhibited in the presence of a given antibiotic without a DNA segment that confers resistance thereto.

TcR - the tetracycline resistance-conferring gene; also used to denote the tetracycline-resistant phenotype.

Transcription Terminator - a DNA sequence that signals the termination of transcription of DNA by RNA polymerase.

Transfectant - a recipient host cell that has undergone transformation by phage DNA or by DNA packaged into a phage particle.

Transformant - a recipient host cell that has undergone transformation.

Transformation - the introduction of DNA into a recipient host cell that changes the genotype and results in a change in the recipient cell.

Translational activating sequence - a regulatory DNA sequence that, when transcribed into mRNA, promotes translation of mRNA into protein.

tRNA - transfer ribonucleic acid.

The restriction site and function maps presented in the accompanying drawings are approximate representations of the recombinant DNA vectors discussed herein. The restriction site information is not exhaustive; therefore, there may be more restriction sites of a given type on the vector than actually shown on the map.

Figure 1. Acyltransferase reactions.

Figure 2. The beta-lactam biosynthetic pathway.

Figure 3. A restriction site and function map of plasmid pOGO4.

Figure 4 - A restriction site and function map of plasmid pUT715.

Figure 5 - A restriction site and function map of plasmid pLC2.

Figure 6 - A restriction site and function map of plasmid pKC787.

Figure 7 - A restriction site and function map of plasmid pRH5.

Figure 8 - A restriction site and function map of plasmid pOW1061.

Figure 9 - A restriction site and function map of plasmid pOW1062.

Figure 10 - A restriction site and function map of plasmid pOW1063.

Figure 11 - A restriction site and function map of plasmid pOW1064.

Figure 12 - A restriction site and function map of plasmid pOW1065.

Figure 13 - A restriction site and function map of plasmid pPS95.

The present invention comprises an isolated DNA compound that comprises a DNA sequence encoding isopenicillin N:acyl CoA acyltransferase activity of Aspergillus nidulans. The present invention also comprises DNA compounds and recombinant DNA cloning and expression vectors that encode the acyltransferase activity of Aspergillus nidulans. The sequence of the Aspergillus nidulans acyltransferase-encoding DNA is depicted below, together with the portions of the DNA that flank the 5′ and 3′ ends of the coding region in the A. nidulans genome. In the depiction, only the coding strand of the double-stranded DNA molecule is shown, and the DNA is depicted from left to right in the 5′ → 3′ orientation. The nucleotide sequence is numbered; the numbers appear to the left of the DNA sequence.

```
  1 CTGCAGAATT GCACAGGCCC TTTTCGCTCC ACCCGCAGCG CCACGAATCC
 51 GGTTGGCATC GGCGCGATGT CACCTGCAAT GGAACGGCGA TAGGATCTTG
101 ATGCTCACCA GCCAATCACA GGTTCTCCAA GAAGCACTCG GGATCCACGG
151 GTTCCGTCTC AGTAGCGGCT GCGTACACGA TCCTAACCTA AAAGAACCAC
201 AGGCCAAGCC CACATGCATA GCATGTTGAT TTCCCTGGCC CGGCGCCGGC
251 CGAACCTTGG TCTAGGGCTT TGTGCGCTCT GTCGATTCCG TGTCAAAACT
301 GGACCAGGCA GCAGGAACAT GTCTGTATTC TAGATCTCAT CGGTACTTGC
351 CGCCAACTCA CCCAAATCGC GATCTCGAAT GCACCTATCT AGCTAGCCAC
401 CCTGTTCAAT ATTTTCTACT GGACGCTCCG AAGAAAAATG CTTCACGTAA
451 CTTGCCAAGG TACCCCCTCC GAAGTAAGTC CATACCCGAA TGTATATTTG
```

501 CCCATATTTA ACGCCTATAC TTCCAGATCG GCTATCACCA TGGCTCTGCT
551 GCCAAAGGCG AGATTGCGAA AGCCATTGAC TTCGCAACTG GCCTCATTCA
601 TGGCAAAACA AAAAAGACAC AGGCGGAGCT TGAACAGCTC CTCAGGGAGT
651 TGGAGCAGGT GATGAAACAG CGCTGGCCGA GATACTATGA GGAAATCTGC
701 GGTATGTTTA CCTACGTTCT TTACTTGCTG AATCACCCCG TTGACGGAAT
751 TTTCAGGAAT CGCAAAGGGT GCGGAACGCG AAGTATCGGA GATTGTCATG
801 CTCAACACTC GTACGGAATT CGCGTACGGG CTCGTAGAAG CCCGGGACGG
851 GTGCACCACT GTTTACTGCA AAACCCCCAA TGGAGCGCTA CAGGGCCAGA
901 ACTGGGACGT AGGTTATTAG ATCCACGGTC TGCTATCTAT TTTCCTTGCT
951 AACCGCCATT CCGGCAGTTC TTCACCGCAA CCAAAGAAAA CTTGATCCAG
1001 TTAACAATTT GTCAGCCGGG TCTACCCACT ATCAAAATGA TTACAGAAGC
1051 TGGTATCATT GGCAAAGTGG GTTTCAACAG TGCTGGTGTC GCTGTCAATT
1101 ACAATGCACT ACACCTACAT GGCCTCCGTC CCACTGGCCT CCCCTCGCAT
1151 CTCGCGCTGC GCATGGCCCT CGAAAGTACA TCTCCGTCTG AGGCGTATGA
1201 AAAAATCGTC TCGCAAGGGG GCATGGCGGC TAGCGCGTTC ATCATGGTGG
1251 GCAACGCACA CGAGGCCTAC GGGCTAGAGT TCTCGCCCAT CAGCTTGTGC
1301 AAGCAAGTTG CTGACACCAA TGGGCGGATA GTGCATACGA ACCACTGCCT
1351 CCTCAACCAT GGGCCATCGG CGCAAGAGCT TAATCCCCTG CCGGACTCGT
1401 GGAGCCGCCA CGGGCGGATG GAACATCTCC TCTCTGGTTT TGACGGCACG
1451 AAGGAGGCAT TTGCGAAGTT GTGGGAGGAC GAAGACAACT ACCCTCTCTC
1501 GATCTGCCGG GCATATAAGG AAGGGAAAAG TAGAGGCTCC ACTCTTTTCA
1551 ACATCGTCTT CGATCATGTG GGCCGGAAGG CAACAGTGCG GCTGGGCCGG
1601 CCCAATAACC CTGATGAGAC CTTTGTCATG ACCTTTAGCA ATCTGGATAC
1651 CAAGTCCGCG ATCCAAGCCA ACATTTGACC AATATCTCTT TCCAACCGAT
1701 GCCTTCTGCA GTCTTCTCCA GCCATAGCGT ACTACACCGT GAACACTTCT
1751 ATGTATCTTG ACAACTAGAT ACAAGTCAAA AGGCGAATCA AATAAAGTCG
1801 CATGTTTGGG AGAATGCACG CCAGAGTGAA ATGCTGAATC CTGTTGGCTT
1851 TCACAACCAG GTGCCTAAAG TATAGCAATG CAACGAAGGC ATGCCTAGTC
1901 TAGCTTCTTT CCCTAAACAT GCTTTAAATC TAATACGAGA ATTATTTAAA
1951 GCATTCCAGA ACTACTACTA ACTTCCTATT ACCCTGGAGT TTCCTGTTTC
2001 GGAAGGGTTG ACCTCTCAAT CCTTCCTAAC CTTGTGAGTC TAGAGTAAGC

| | | | | | |
|---|---|---|---|---|---|
| 2051 | TCTGCAATCT | TGTTTAACCT | ACCTAGGACT | CACGAAGACT | CCCTCAGTAT |
| 2101 | GAAGTTGATA | AAGGTTCTTT | GTGCTCCGAG | CCCGCAAGGG | AAATCAGTGC |
| 2151 | TGAGATATAC | TGGCTGGGCT | CTATATGTTG | TAGAGTCAAG | TGTTGACAAT |
| 2201 | TTGAATCCTC | GCTAATGTCA | CGGCTCTGCA | AGCCGGGATC | CCGAGTTGGT |
| 2251 | AAAGCATGCA | ACAGCAAGCC | CCATTTTATC | TACATATTAG | CTACGATTAT |
| 2301 | CCTAATCATT | GCTTTTAGAA | TAATCTCTAA | ATTTGGAGCC | AGCAGGTCAC |
| 2351 | AGAGCTCACT | CTAGGCACCC | CGGGCACAGT | GCTCGAAATA | GCGTCGTGCC |
| 2401 | GTTATCCAGC | TTCATACATT | GGTGTAAAAG | AAGAATCGAG | CCATTTCTGG |
| 2451 | CCACCCATCT | GATAAAGTTA | GGCCTAGCTC | CGTGAAGACA | TTCCACAAAA |
| 2501 | ATCTCTCCAT | GAACATAATT | CTTCTCAGTG | CTTAGTTTCT | TGAACATGAG |
| 2551 | CTACAGAATG | GTTTTTTGAA | TATTTCAATT | GGCTTTCCAC | CATAATATAT |
| 2601 | GATTATAGTT | ACTAATTAGA | CTACTTCTAT | AGCAAAGCTT | |

wherein A is a deoxyadenyl residue, G is a deoxyguanyl residue, C is a deoxycytidyl residue, and T is a thymidyl residue.

Following is the amino acid sequence encoded by the preceding DNA sequence. The DNA sequence preceding the methionine codon at position 438 is 5′ untranslated sequence. The first exon consists of bases 438-473, the second is from 527 to 701, the third is from 757 to 908, and the fourth is from 968 to 1675. The sequence from 1676 to the end is 3′ untranslated region. The first and last base of each intron is marked by an asterisk.

1 CTGCAGAATTGCACAGGCCCTTTTCGCTCCACCCGCAGCGCCACGAATCCGGTTGGCATC 60

61 GGCGCGATGTCACCTGCAATGGAACGGCGATAGGATCTTGATGCTCACCAGCCAATCACA 120

121 GGTTCTCCAAGAAGCACTCGGGATCCACGGGTTCCGTCTCAGTAGCGGCTGCGTACACGA 180

181 TCCTAACCTAAAAGAACCACAGGCCAAGCCCACATGCATAGCATGTTGATTTCCCTGGCC 240

241 CGGCGCCGGCCGAACCTTGGTCTAGGGCTTTGTGCGCTCTGTCGATTCCGTGTCAAAACT 300

301 GGACCAGGCAGCAGGAACATGTCTGTATTCTAGATCTCATCGGTACTTGCCGCCAACTCA 360

361 CCCAAATCGCGATCTCGAATGCACCTATCTAGCTAGCCACCCTGTTCAATATTTTCTACT 420

*

421 GGACGCTCCGAAGAAAAATGCTTCACGTAACTTGCCAAGGTACCCCCTCCGAAGTAAGTC 480

MetLeuHisValThrCysGlnGlyThrProSerGlu

*

481 CATACCCGAATGTATATTTGCCCATATTTAACGCCTATACTTCCAGATCGGCTATCACCA 540

IleGlyTyrHisHis

541 TGGCTCTGCTGCCAAAGGCGAGATTGCGAAAGCCATTGACTTCGCAACTGGCCTCATTCA 600

GlySerAlaAlaLysGlyGluIleAlaLysAlaIleAspPheAlaThrGlyLeuIleHis

601 TGGCAAAACAAAAAAGACACAGGCGGAGCTTGAACAGCTCCTCAGGGAGTTGGAGCAGGT 660

GlyLysThrLysLysThrGlnAlaGluLeuGluGlnLeuLeuArgGluLeuGluGlnVal

*

661 GATGAAACAGCGCTGGCCGAGATACTATGAGGAAATCTGCGGTATGTTTACCTACGTTCT 720

MetLysGlnArgTrpProArgTyrTyrGluGluIleCysGly

*

721 TTACTTGCTGAATCACCCCGTTGACGGAATTTTCAGGAATCGCAAAGGGTGCGGAACGCG 780

IleAlaLysGlyAlaGluArgGlu

781 AAGTATCGGAGATTGTCATGCTCAACACTCGTACGGAATTCGCGTACGGGCTCGTAGAAG 840

ValSerGluIleValMetLeuAsnThrArgThrGluPheAlaTyrGlyLeuValGluAla

841 CCCGGGACGGGTGCACCACTGTTTACTGCAAAACCCCCAATGGAGCGCTACAGGGCCAGA 900

ArgAspGlyCysThrThrValTyrCysLysThrProAsnGlyAlaLeuGlnGlyGlnAsn

*

901 ACTGGGACGTAGGTTATTAGATCCACGGTCTGCTATCTATTTTCCTTGCTAACCGCCATT 960

TrpAsp

*

961 CCGGCAGTTCTTCACCGCAACCAAAGAAAACTTGATCCAGTTAACAATTTGTCAGCCGGG
PhePheThrAlaThrLysGluAsnLeuIleGlnLeuThrIleCysGlnProGly 1020

1021 TCTACCCACTATCAAAATGATTACAGAAGCTGGTATCATTGGCAAAGTGGGTTTCAACAG
LeuProThrIleLysMetIleThrGluAlaGlyIleIleGlyLysValGlyPheAsnSer 1080

1081 TGCTGGTGTCGCTGTCAATTACAATGCACTACACCTACATGGCCTCCGTCCCACTGGCCT
AlaGlyValAlaValAsnTyrAsnAlaLeuHisLeuHisGlyLenArgProThrGlyLeu 1140

1141 CCCCTCGCATCTCGCGCTGCGCATGGCCCTCGAAAGTACATCTCCGTCTGAGGCGTATGA
ProSerHisLeuAlaLeuArgMetAlaLeuGluSerThrSerProSerGluAlaTyrGlu 1200

1201 AAAAATCGTCTCGCAAGGGGGCATGGCGGCTAGCGCGTTCATCATGGTGGGCAACGCACA
LysIleValSerGlnGlyGlyMetAlaAlaSerAlaPheIleMetValGlyAsnAlaHis 1260

1261 CGAGGCCTACGGGCTAGAGTTCTCGCCCATCAGCTTGTGCAAGCAAGTTGCTGACACCAA
GluAlaTyrGlyLeuGluPheSerProIleSerLeuCysLysGlnValAlaAspThrAsn 1320

1321 TGGGCGGATAGTGCATACGAACCACTGCCTCCTCAACCATGGGCCATCGGCGCAAGAGCT
GlyArgIleValHisThrAsnHisCysLeuLeuAsnHisGlyProSerAlaGlnGluLeu 1380

1381 TAATCCCCTGCCGGACTCGTGGAGCCGCCACGGGCGGATGGAACATCTCCTCTCTGGTTT
AsnProLeuProAspSerTrpSerArgHisGlyArgMetGluHisLeuLeuSerGlyPhe 1440

1441 TGACGGCACGAAGGAGGCATTTGCGAAGTTGTGGGAGGACGAAGACAACTACCCTCTCTC
AspGlyThrLysGluAlaPheAlaLysLeuTrpGluAspGluAspAsnTyrProLeuSer 1500

1501 GATCTGCCGGGCATATAAGGAAGGGAAAAGTAGAGGCTCCACTCTTTTCAACATCGTCTT
IleCysArgAlaTyrLysGluGlyLysSerArgGlySerThrLeuPheAsnIleValPhe 1560

1561 CGATCATGTGGGCCGGAAGGCAACAGTGCGGCTGGGCCGGCCCAATAACCCTGATGAGAC
AspHisValGlyArgLysAlaThrValArgLeuGlyArgProAsnAsnProAspGluThr 1620

1621 CTTTGTCATGACCTTTAGCAATCTGGATACCAAGTCCGCGATCCAAGCCAACATTTGACC
PheValMetThrPheSerAsnLeuAspThrLysSerAlaIleGlnAlaAsnIleEnd 1680

1681 AATATCTCTTTCCAACCGATGCCTTCTGCAGTCTTCTCCAGCCATAGCGTACTACACCGT 1740

1741 GAACACTTCTATGTATCTTGACAACTAGATACAAGTCAAAAGGCGAATCAAATAAAGTCG 1800

1801 CATGTTTGGGAGAATGCACGCCAGAGTGAAATGCTGAATCCTGTTGGCTTTCACAACCAG 1860

1861 GTGCCTAAAGTATAGCAATGCAACGAAGGCATGCCTAGTCTAGCTTCTTTCCCTAAACAT 1920

1921 GCTTTAAATCTAATACGAGAATTATTTAAAGCATTCCAGAACTACTACTAACTTCCTATT 1980

1981 ACCCTGGAGTTTCCTGTTTCGGAAGGGTTGACCTCTCAATCCTTCCTAACCTTGTGAGTC 2040

2041 TAGAGTAAGCTCTGCAATCTTGTTTAACCTACCTAGGACTCACGAAGACTCCCTCAGTAT 2100

2101 GAAGTTGATAAAGGTTCTTTGTGCTCCGAGCCCGCAAGGGAAATCAGTGCTGAGATATAC 2160

2161 TGGCTGGGCTCTATATGTTGTAGAGTCAAGTGTTGACAATTTGAATCCTCGCTAATGTCA 2220

2221 CGGCTCTGCAAGCCGGGATCCCGAGTTGGTAAAGCATGCAACAGCAAGCCCCATTTTATC 2280

2281 TACATATTAGCTACGATTATCCTAATCATTGCTTTTAGAATAATCTCTAAATTTGGAGCC 2340

2341 AGCAGGTCACAGAGCTCACTCTAGGCACCCCGGGCACAGTGCTCGAAATAGCGTCGTGCC 2400

2401 GTTATCCAGCTTCATACATTGGTGTAAAAGAAGAATCGAGCCATTTCTGGCCACCCATCT 2460

2461 GATAAAGTTAGGCCTAGCTCCGTGAAGACATTCCACAAAAATCTCTCCATGAACATAATT 2520

2521 CTTCTCAGTGCTTAGTTTCTTGAACATGAGCTACAGAATGGTTTTTTGAATATTTCAATT 2580

2581 GGCTTTCCACCATAATATATGATTATAGTTACTAATTAGACTACTTCTATAGCAAAGCTT 2640

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

Those skilled in the art will recognize that the DNA sequence depicted above is an important part of the present invention. The above sequence can be conventionally synthesized by the modified phosphotriester method using fully protected deoxyribonucleotide building blocks. Such synthetic methods are well known in the art and can be carried out in substantial accordance with the procedure of Itakura et al., 1977, Science 198:1056 and Crea et al., 1978, Proc. Nat. Acad. Sci. USA 75:5765. In addition, an especially preferred method is disclosed in Hsiung et al., 1983, Nucleic Acid Research 11:3227 and Narang et al., 1980, Methods in Enzymology 68:90. In addition to the manual procedures referenced above, the DNA sequence can be synthesized using automated DNA synthesizers, such as the ABS (Applied Biosystems, 850 Lincoln Centre Drive, Foster City, CA 94404) 380A DNA Synthesizer. The DNA sequence can also be generated by the polymerase chain reaction. See, e.g., U.S. Patents Serial Nos. 4,800,159 and 4,683,202 and European Patent Publication No. 0258017, published March 2, 1987.

The amino acid residue sequence of acyltransferase enzyme depicted above can be encoded by a multitude of different DNA sequences because most of the amino acid residues are encoded by more than one DNA triplet. Because these alternate DNA sequences would encode the same amino acid residue sequence of the present invention, the present invention further comprises these alternate sequences.

The present invention comprises DNA compounds and recombinant DNA cloning and expression vectors that encode the acyltransferase activity of Aspergillus nidulans. The acyltransferase activity encoding DNA compounds of the present invention were isolated from a strain of A. nidulans. A genomic library of the total genomic DNA of the A. nidulans strain was constructed and examined for the presence of sequences homologous to a deoxyribooligonucleotide probe. This probe was constructed in accordance with information obtained about the amino-terminal amino acid sequence of the Penicillium chrysogenum acyltransferase and with knowledge of the genetic code. DNA sequencing revealed the open reading frame of the A. nidulans acyltransferase.

The Aspergillus nidulans acyltransferase gene can be isolated on an ~2.1 kb PvuI-SphI fragment derived from plasmid pOGO4, which has been deposited with the Northern Regional Research Laboratories (NRRL), Peoria, IL 61604, in E. coli K12 JM109 under the accession number NRRL B-18171. A restriction site and function map of plasmid pOGO4

is presented in Figure 3 of the accompanying drawings.

Plasmid pOGO4 serves as useful starting material for the construction of other expression vectors of the invention. These vectors are especially useful in a method for producing acyltransferase activity in a recombinant host cell, said method comprising: (1) transforming said host cell with a recombinant DNA expression vector that comprises: (a) a promoter and translational activating sequence; and (b) a DNA sequence that encodes acyltransferase activity and is positioned for expression from said promoter and translational activating sequence; and (2) culturing said host cell transformed in step (1) under conditions that allow for expression of said acyltransferase activity.

Plasmid pOGO4 can be isolated from E. coli K12 JM109/pOGO4 by the procedure described in Example 1. Plasmid pOGO4 contains the intact Aspergillus nidulans acyltransferase gene, which can be isolated, for example, from the plasmid on an ~2.1 kb PvuI-SphI restriction fragment.

The acyltransferase expression vectors of the present invention are not limited to a particular selectable marker. Those skilled in the art recognize that many selectable markers are suitable for use on acyltransferase expression vectors. Such selectable markers include, for example, genes that confer kanamycin resistance, genes that confer chloramphenicol resistance, or other antibiotic resistance-conferring genes. In Aspergillus and Penicillium, the useful markers are the acetamidase gene (amdS) and the phleomycin resistance gene (phlR). Expression vectors containing the ampicillin resistance gene are less useful because the gene encodes beta-lactamase, which, if expressed, will degrade the reaction product.

In prokaryotic systems, the introns of the gene (as delineated on the DNA sequence supra) may be removed using the techniques of restriction enzyme digestion and site-specific mutagenesis if necessary for expression. The gene may also be used as a hybridization probe to isolate a cDNA clone which necessarily has no introns and can therefore be expressed in prokaryotes. All the techniques required to generate this array of vectors and transformed microorganisms are familiar to one skilled in the art. All such embodiments are within the scope of the present invention.

The present invention is not limited to the particular vectors exemplified herein. Instead, the present invention comprises DNA compounds that encode the acyltransferase activity of Aspergillus nidulans. The DNA compounds of the present invention can be used to construct expression vectors that drive expression of acyltransferase activity in any host cell in which the expression vector replicates or integrates and in which the promoter and translational activating sequence are functional.

Therefore, the present invention comprises any E. coli expression plasmid or vector that drives expression of acyltransferase protein in E. coli. Thus, the present invention comprises expression vectors that drive expression of acyltransferase protein and utilize a replicon functional in E. coli, such as, for example, a replicon from such plasmids as pBR322, pACYC184, F, ColV-K94, R1, R6-5, or R100. Nor is the present invention solely limited to plasmid vectors, for the present invention also comprises expression vectors that express the acyltransferase protein and utilize integration or viral replication to provide for replication and maintenance in the host cell.

The present invention is not limited to a particular promoter and translational activating sequence to drive expression of the acyltransferase gene. The present invention comprises the use of any promoter and translational activating sequence that function in E. coli and are used to express acyltransferase protein in E. coli. Many promoter and translational activating sequences functional in E. coli are known and are suitable for driving expression of acyltransferase protein in E. coli. Such transcriptional and translational activating sequences include, but are not limited to, the lpp, lac, trp, tac, λpL, and λpR promoter and translational activating sequences.

In addition, transcriptional and translational activating sequences from other organisms can be ligated to the present acyltransferase activity-encoding DNA compounds to form expression vectors that drive expression of acyltransferase activity in host cells in which the activating sequence functions. Vectors that drive expression of acyltransferase activity in host cells other than E. coli are also useful, especially for purposes of increasing the antibiotic producing ability and efficiency of a given organism.

A variety of organisms produce β-lactam antibiotics. The following Table presents a non-comprehensive list of β-lactam antibiotic-producing organisms.

TABLE I

β-Lactam Antibiotic-Producing Organisms

| Organism | Antibiotic |
| --- | --- |
| Agrobacterium | various β-lactams |
| Arachnomyces minimus | penicillins and cephalosporins |
| Anixiopsis peruviana | penicillins and cephalosporins |
| Aspergillus | penicillins |
| Cephalosporium acremonium, purpurascens, polyaleurum, chrysogenum, curtipes | penicillins and cephalosporins |
| Chromobacterium | various β-lactams |
| Emericellopsis terricola, minima, synnematicola, glabra, mirabilis, salmosynnemata | penicillins and cephalosporins |
| Flavobacterium | various β-lactams |
| Gluconobacter | various β-lactams |
| Nocardia lactamadurans | cephamycin C |
| Nocardia uniformis | nocardicin |
| Paecilomyces carneus, persicinus | penicillins and cephalosporins |

TABLE I continued

| | |
|---|---|
| Penicillium chrysogenum | various penicillins and other β-lactams |
| Serratia | various β-lactams |
| Spiroidium fuscum | penicillins and cephalosporins |
| Streptomyces | |
| antibioticus | clavulanic acid |
| argenteolus | asparenomycin A, MM 4550, and MM 13902 |
| cattleya | thienamycin |
| chartreusis | SF 1623 and cephamycin A and B |
| cinnamonensis | cephamycin A and B |
| clavuligerus | PA-32413-I, cephamycin C, A16886A, penicillins, cephalosporins, clavulanic acid, and other clavams |
| fimbriatus | cephamycin A and B |
| flavovirens | MM 4550 and MM 13902 |
| flavus | MM 4550 and MM 13902 |
| fulvoviridis | MM 4550 and MM 13902 |
| griseus | cephamycin A and B and carpetimycin A and B |
| halstedi | cephamycin A and B |
| heteromorphus | C2081X and cephamycin A and B |
| hygroscopicus | deacetoxy-cephalosporin C |
| lipmanii | cephamycin, penicillin N, 7-methoxycephalosporin C, A16884, MM4550, MM13902 |
| olivaceus | epithienamycin F, MM 4550, and MM 13902 |
| panayensis | C2081X and cephamycin A and B |
| pluracidomyceticus | pluracidomycin A |
| rochei | cephamycin A and B |
| sioyaensis | MM 4550 and MM 13902 |
| sp. OA-6129 | OA-6129A |
| sp. KC-6643 | carpetimycin A |
| tokunomensis | asparenomycin A |
| viridochromogenes | cephamycin A and B |
| wadayamensis | WS-3442-D |

Some of the foregoing β-lactam antibiotic-producing organisms are used in the pharmaceutical industry for purposes of antibiotic production. The antibiotic-producing ability of these organisms can be increased and made more efficient by increasing the intracellular concentration of the antibiotic biosynthetic enzymes during the fermentation. The acyltransferase activity-encoding DNA compounds of the present invention can be used to construct expression vectors that, when transformed into the appropriate host cell, increase the intracellular concentration of acyltransferase activity

of the transformed host cell and thereby increase the antibiotic-producing ability and efficiency of that cell, provided that the host cell produces a β-lactam antibiotic via an intermediate reaction involving acyltransferase activity.

A vector that will increase the intracellular concentration of acyltransferase activity of a given host cell into which the vector is transformed requires the following elements: 1) an acyltransferase activity encoding DNA compound of the present invention; and 2) a promoter and translational activating sequence that not only function in the host cell to be transformed, but also are positioned in the correct orientation and position to drive expression of the acyltransferase activity encoding DNA. Of course, stable transformants can only be obtained if the vector replicates, either as an extrachromosomal element or integrated in the genomic DNA, in the host cell. Thus, a preferred vector contains sequences that specifically direct replication or integration of the vector in the host cell. However, the presence of such specific replication or integration sequences is not absolutely required, as non-specific integration may occur when DNA is introduced into a host cell. An acyltransferase expression vector could also comprise an antibiotic resistance conferring gene or some other element that provides a means of selecting for host cells which contain the vector, but such selectable elements may neither be necessary nor desired when the vector integrates into the chromosomal DNA of the host cell.

By providing the coding sequence of the acyltransferase gene of Aspergillus nidulans, the present invention provides acyltransferase expression vectors for any organism susceptible to transformation. The E. coli acyltransferase expression vectors described above illustrate the wide variety of expression vectors of the present invention. However, many of the preferred vectors of the invention are designed to drive expression of acyltransferase in a β-lactam (including penicillins and cephalosporins) antibiotic-producing cell.

The Penicillium vectors of the invention are illustrative of the vectors provided by the present invention that can be used to increase the yield of antibiotic from such a β-lactam antibiotic-producing cell or, in the case of vectors used to disrupt acyltransferase production, to alter the antibiotic normally produced by the cell.

Acyltransferase expression vectors that contain the acetamidase gene or the phleomycin resistance gene are particularly useful as vectors for inserting genes into Penicillium chrysogenum because no special recipient strain, such as an auxotroph, need be constructed, owing to the natural inability of P. chrysogenum to grow on acetamide as sole nitrogen source or in the presence of phleomycin. Transformation systems based on complementation of auxotrophic markers by a gene in the transforming plasmid do not share this advantage. Frequently, pleiotropic mutations are associated with the introduction of an auxotrophic marker into a P. chrysogenum strain highly developed for penicillin production. Such mutations usually result in lower penicillin production (MacDonald et al., 1963, J. Gen. Microbiol. 33: 365-374). The vectors described above and in the Examples are merely illustrative of the wide variety of acyltransferase expression vectors provided by the present invention.

The acyltransferase expression vectors of the present invention are useful for increasing the intracellular concentration of acyltransferase activity in many cells, especially β-lactam antibiotic-producing cells. Plasmid pOGO4 comprises the coding sequence of the acyltransferase gene of Aspergillus nidulans. Fragments derived from plasmid pOGO4 can be used to construct vectors for increasing the copy number of the acyltransferase gene and thus for increasing the intracellular concentration of the enzyme. Because the acyltransferase coding sequence of the invention was isolated from an Aspergillus host cell, the acyltransferase coding sequence is particularly well-suited for use in expression vectors designed to drive high-level expression of acyltransferase activity in Aspergillus host cells. In addition, since many Aspergillus genes have been readily expressed in Penicillium, high level expression of the Aspergillus acyltransferase gene is likely to occur in Penicillium.

The Aspergillus nidulans acyltransferase coding sequence of the invention can also be put under the control of transcription and translation activating sequences derived from strains of Penicillium, as well as from Cephalosporium, or any other host cell to construct a recombinant acyltransferase gene for use in the given organism.

A preferred use of the acyltransferase gene is to generate useful cephalosporins in Aspergillus and Penicillium via disruption or displacement of the acyltransferase gene along with the addition of genes encoding the epimerase (racemase) and expandase enzymes. In Aspergillus, the acyltransferase gene of the Aspergillus genome is disrupted by single or double cross-overs with the cloned gene which has a marker such as an antibiotic resistance gene inserted into it. The cell is then transformed with vector(s) comprising expandase (available on an ~3.0 kb BamHI restriction fragment isolated from plasmid pOW380 (NRRL B-18264)) and epimerase (available on an ~3.6 kb KpnI restriction fragment isolated from plasmid pOW390 (NRRL B-18431)) genes. See U.S. Patent Applications Serial Nos. 07/192,273, filed May 9, 1988 and 07/288,760, filed December 22, 1988 (European Patent Publication Nos. 0341892 and 0377295) for further disclosures concerning those genes. The Examples demonstrate this method.

An analogous method is useful for generating large amounts of cephalosporins in Penicillium. Penicillium is an especially preferred organism to employ because Penicillium has been modified to produce very large amounts of the beta-lactam nucleus, thereby providing large quantities of substrate for conversion to cephalosporins. The Penicillium chrysogenum acyltransferase gene can be isolated using the Aspergillus DNA sequence of the present invention as a hybridization probe. The gene can be isolated from a publicly available strain of Penicillium chrysogenum such as ATCC 10238 (available from the American Type Culture Collection, Rockville, MD 20852).

While inactivation of acyltransferase genes can be achieved by gene disruption or gene displacement, both techniques are most effective in host organisms that contain only one copy of the acyltransferase gene. For those organisms that contain multiple copies of the acyltransferase gene, expression of anti-sense RNA complementary to the acyltransferase mRNA would substantially decrease acyltransferase activity.

Thus a penicillin G-producing fungus can be converted into a cephalosporin-producing fungus, by a method comprising:

(1) transforming a penicillin G-producing fungal cell with a recombinant DNA vector that comprises a DNA sequence derived from an acyltransferase gene;
(2) eliminating acyltransferase gene expression in the fungal cell; and
(3) transforming the fungal cell of step (2) with one or more vectors comprising genes which code for the expression of expandase and epimerase enzymatic activity.

Cephalosporins may be produced by culturing the resultant fungal strain under conditions suitable for cephalosporin production. See U.S. Patents Nos. 3,847,742 and 4,762,786 which are herein incorporated by reference. Also herein incorporated by reference is U.S. Patent Application Serial No. 06/801,523, (European Patent Publication Number 0225 128) which has been allowed and the issue fee has been paid. Such conditions are well-known to one skilled in the art.

Another method for converting a penicillin G-producing fungus into a cephalosporin-producing fungus comprises:

transforming a penicillin G-producing fungal cell with one or more recombinant DNA vectors that comprise genes encoding the enzymes expandase and epimerase and a DNA sequence which can be transcribed to form an RNA molecule complementary to the acyltransferase gene mRNA.

Cephalosporins may be produced by culturing the resultant fungal strain under conditions where such acyltransferase anti-sense RNA is produced by transcription and under conditions suitable for cephalosporin production as described above. Such conditions are well-known to one skilled in the art.

Large amounts of penicillin N, a compound that is difficult to produce chemically, can also be produced. This is accomplished by first eliminating the expression of the acyltransferase gene of a penicillin G-producing fungus by either recombination or anti-sense RNA, followed by introduction of an epimerase gene into such a fungus. Thus there are two methods for converting a penicillin G-producing fungus into a penicillin N-producing fungus, the first method comprising:

transforming a penicillin G-producing fungal cell with one or more recombinant DNA vectors that comprise a gene encoding the enzyme epimerase and a DNA sequence which can be transcribed to form an RNA molecule complementary to the acyltransferase gene mRNA.

Penicillin N may be produced by culturing the resultant fungal cell under conditions where such acyltransferase complementary RNA molecule is produced by transcription and under conditions suitable for antibiotic production as described supra. Such methods are well-known to one skilled in the art.

The second method for converting a penicillin G-producing fungus into a penicillin N-producing fungus comprises:

(1) transforming a penicillin G-producing fungal cell with a recombinant DNA vector that comprises a DNA sequence derived from an acyltransferase gene;
(2) eliminating acyltransferase gene expression in the fungal cell; and
(3) transforming the fungal cell of step (2) with a vector comprising a gene encoding epimerase enzymatic activity.

Penicillin N may be produced by culturing the resultant fungal cell under conditions suitable for antibiotic production as described supra. Such conditions are well-known to one skilled in the art.

The following Examples are provided to further illustrate and exemplify, but do not limit the scope of, the present invention.

## Example 1

### Source of the Aspergillus acyltransferase gene

#### A. Culture of E. coli K12 JM109/pOGO4

A lyophil of E. coli K12 JM109/pOGO4 can be obtained from the Northern Regional Research Laboratories (NRRL), Peoria, IL 61604, under the accession number NRRL B-18171, (date of deposit: February 6, 1987) and used directly as the "culture" in the process described below.

One liter of TY broth (8 g tryptone, 5 g NaCl, and 5 g yeast extract per liter) containing 100 µg/ml ampicillin was inoculated with a culture of E. coli K12 JM109/pOGO4 and incubated with aeration at 37°C overnight (15-18 hours). The resulting culture was used as a source of plasmid pOGO4 (Figure 3).

B. Isolation of Plasmid pOGO4

The culture prepared in Example 1A was centrifuged at 5200 rpm in a Sorvall GSA rotor (DuPont, Instrument Products, Biomedical Division, Newtown, CT 06470) for 10 minutes at 4°C to pellet the cells. The resulting supernatant was discarded. The cell pellet was resuspended in 28 ml of a solution of 25% sucrose and 50 mM ethylenediamine tetraacetic acid (EDTA). About 1 ml of a solution of 20 mg/ml lysozyme in 50% glycerol and 0.25 M Tris-HCl (Tris (hydroxymethyl) aminomethane hydrochloride), pH = 8.0, and about 1.5 ml of 0.5 M EDTA, pH = 8.0, were added to and mixed with the cell suspension. The resulting mixture was incubated on ice for 15 minutes. Three ml of lysing solution (prepared by mixing 3 ml of 10% Triton X-100; 75 ml of 0.25 M EDTA, pH = 8.0; and 7 ml of water) were added to the lysozyme-treated cells with gentle mixing. The resulting solution was incubated on ice for another 15 minutes.

The cellular debris was removed from the solution by centrifugation at 17,000 rpm in a Sorvall SS34 rotor (DuPont, Instrument Products, Biomedical Division, Newtown, CT 06470) for about 45 minutes at 4°C. About 28.6 g of CsCl and ~1 ml of a 5 mg/ml ethidium bromide solution were added to the ~30 ml of supernatant. Then, the volume was adjusted to 40 ml with water and the solution decanted into an ultracentrifuge tube. The tube was sealed, and the solution was centrifuged at 49,500 rpm in a Ti70 rotor (Beckman, 7360 N. Lincoln Avenue, Lincolnwood, IL 60646) for ~18 hours. The resulting plasmid band, visualized with ultraviolet light, was isolated, extracted with CsCl-saturated isopropanol to remove the ethidium bromide, and dialysed against three changes of ~20 volumes of TE buffer (10 mM Tris-HCl pH = 7.5, and 1 mM EDTA). The dialysate was collected; then, two volumes of ethanol and 0.05 volumes of 3 M sodium acetate solution were added. The ethanol mixture was cooled to -20°C, and the plasmid DNA was pelleted by centrifugation at 10,000 rpm in an SS34 rotor for 30 minutes at -10°C. The resulting pellet was resuspended in ~1 ml of TE buffer and then extracted with an equal volume of a phenol:chloroform mixture (1:1, v/v). The DNA in the aqueous phase was recovered by the addition of 0.1 volume of 3 M sodium acetate and 2 volumes of ethanol, followed by incubation at -20°C for ~30 minutes and centrifugation at 15,000 rpm in an SS34 rotor for 20 minutes. The resulting DNA pellet was rinsed first with 70% ethanol and then with 100% ethanol and dried.

The ~1.5 mg of plasmid pOGO4 DNA obtained by this procedure was suspended in 1.5 ml of 0.1X TE buffer and stored at -20°C. A restriction site and function map of plasmid pOGO4 is presented in Figure 3 of the accompanying drawings.

Example 2

Construction of intermediate plasmid pRH5

A. Source of phleomycin and a plasmid containing a phleomycin resistance gene

Phleomycin was purchased from Cayla, Avenue De Larrieu, 31094 Toulouse, Cedex, France. Plasmid pUT715 which contains a Streptoalloteichus hindustanus phleomycin resistance gene juxtaposed to the 3′ regulatory DNA sequence of the Aspergillus nidulans trpC gene, was also obtained from Cayla. A restriction map and function map of pUT715 is presented in Figure 4 of the accompanying drawings.

B. Source of the Penicillium chrysogenum IPNS promoter

The promoter of the IPNS gene is located within the approximately 0.9 kb of DNA immediately upstream of the Penicillium chrysogenum IPNS gene, bounded by the restriction sites EcoRI and NcoI, in plasmid pLC2 (pXL2 in Carr et al., Gene 48:257-266 (1986)). Plasmid pLC2 may be obtained from the American Type Culture Collection (Rockville, MD 20852) under accession number ATCC 53334 (date of deposit: November 20, 1985) and isolated in substantial accordance with the procedure of Example 1. A restriction map and function map of plasmid pLC2 is presented in Figure 5 of the accompanying drawings. The 0.9 kb EcoRI-NcoI restriction fragment containing the IPNS promoter was isolated from plasmid pLC2 as described below.

The plasmid pLC2 was digested to completion with EcoRI and NcoI as follows. Five µl (~5 µg) of plasmid pLC2 DNA as prepared above are mixed with 38 µl $H_2O$, 5 µl of 10X EcoRI and NcoI restriction buffer (500 mM Tris-HCl (pH 8.0), 100 mM $MgCl_2$, and 1 M NaCl), and 1 µl each of restriction enzymes EcoRI and NcoI (~8 units, Bethesda Research Laboratories, Inc., P.O. Box 577, Gaithersburg, MD 20760). The reaction mixture was incubated for 2 hours at 37°C. The digestion products were then electrophoresed on a 1% agarose gel until the desired ~0.9 kb EcoRI-NcoI restriction fragment was clearly separated from the other digestion products. The electrophoresed DNA was visualized by staining the gel in a dilute solution (0.5 µg/ml) of ethidium bromide and exposing the stained gel to long-wave UV light. After the fragments were located, a small slit was made in the gel in front of the ~0.9 kb fragment, and a piece of Schleicher and Schuell (Keene, NH 03431) DEAE membrane was placed in the slit. Upon further electrophoresis, the DNA non-covalently bound to the DEAE membrane. After the desired fragment was bound to the DEAE membrane, the membrane

was removed and rinsed with low salt buffer (100 mM NaCl; 0.1 mM EDTA; and 20 mM Tris-HCl, pH 8). Next, the membrane was placed in a small tube and immersed in high salt buffer (1 M NaCl; 0.1 mM EDTA; and 20 mM Tris-HCl, pH 8) and then incubated at 65°C for 10 minutes to remove the DNA from the DEAE paper. After the 65°C incubation, the incubation buffer was collected, and the membrane was rinsed with high salt buffer. The rinse solution was pooled with the incubation buffer before collecting the desired DNA fragments.

The volume of the high salt-DNA solution was adjusted so that the NaCl concentration was 0.25 M, and then three volumes of cold, absolute ethanol were added to the solution. The resulting solution was mixed and placed on ice for 10-20 minutes. The solution was then centrifuged at 15,000 rpm in an SS34 rotor for 15 minutes. After another precipitation to remove residual salt, the DNA pellet was rinsed with 70% ethanol, dried, resuspended in 20 µl of TE buffer, and constituted the desired restriction fragment.

C. Source of plasmid pKC787

Plasmid pKC787 provided the apramycin resistance gene for selection of transformants in E. coli and the E. coli origin of DNA replication for the final construction. An E. coli strain carrying plasmid pKC787 may be obtained from the NRRL under accession number NRRL B-18516 (date of deposit: June 30, 1989) and the plasmid isolated in substantial accordance with the teaching of Example 1. A restriction map and function map of plasmid pKC787 is presented in Figure 6 of the accompanying drawings.

D. Construction of plasmid pRH5

Approximately 5 µg of plasmid pKC787 were cut to completion with the restriction enzymes EcoRI and BamHI in substantial accordance with the teaching of Example 2B except that restriction enzyme BamHI was used instead of NcoI. Approximately 5 µg of plasmid pUT715 were cut to completion with the restriction enzymes NcoI and BglII in substantial accordance with the teaching of Example 2B except that restriction enzyme BglII was used instead of EcoRI. Restriction enzymes involved in these reactions were inactivated by extraction with phenol and chloroform, followed by ethanol precipitation. Approximately 0.2 µg of EcoRI/BamHI cut pKC787 DNA, ~0.2 µg of NcoI/BglII cut pUT715 DNA, and ~0.2 µg of the 0.9 kb EcoRI to NcoI fragment containing the Penicillium chrysogenum IPNS promoter from plasmid pLC2 (purification of this fragment was described in section B above) were mixed and ligated in the presence of T4 DNA ligase. The ligation mixture included the DNA fragments, 10 µl of 10X ligase buffer (0.5 M Tris-HCl, pH 7.5 and 100 mM $MgCl_2$), 10 µl 0.1 mM adenosine triphosphate (ATP), 10 µl of 0.1 M DTT, 1 µl T4 DNA ligase (1 unit, Boehringer-Mannheim Biochemicals (BMB), 7941 Castleway Drive, P.O. Box 50816, Indianapolis, IN 46250) and enough water for a total volume of 100 µl. The mixture was incubated at 14°C overnight. The ligation mixture is transformed into E. coli K12 JM109 as follows. Competent E. coli K12 JM109 ("Epicurean Coli™") were purchased from Strategene (3370 Tansy Street, San Diego, CA 92121) and transformed with a ligation reaction mixture comprising plasmid pRH5. The ligated DNA was mixed with 100 µl of competent JM109 cells, incubated for one hour on ice, then incubated for one minute at 42°C, then diluted with 2 ml of fresh TY broth and incubated at 37°C for one hour. Aliquots of the transformation mixture were plated on TY-agar plates containing 100 µg/ml apramycin.

Apramycin resistant transformants were screened for sensitivity to ampicillin. Apramycin resistant, ampicillin sensitive transformants were screened for the presence of a plasmid containing the approximately 2.8 kb EcoRI to BamHI restriction fragment of pKC787, the 0.9 kb EcoRI to NcoI restriction fragment containing the IPNS promoter directly upstream of the approximately 1.3 kb NcoI to BglII fragment from pUT715 which contains the Streptoalloteichus hindustanus phleomycin resistance gene and the trpC 3′ regulatory region from Aspergillus nidulans. A transformant carrying the described plasmid was isolated and the plasmid is designated pRH5. A restriction site and function map of plasmid pRH5 is presented in Figure 7 of the accompanying drawings.

Example 3

A. Isolation of the ~4.8 kb PvuI/ HindIII Fragment from Plasmid pRH5

Plasmid pRH5 (constructed in Example 2) can be isolated from E. coli K12 JM109/pRH5 in accordance with the method of Example 1. Approximately 5 µg of plasmid pRH5 are cut to completion with the restriction enzymes PvuI and HindIII as follows. Five µl (5 µg) of plasmid pRH5 are mixed with 5 µl 10X PvuI buffer (500 mM Tris-HCl (pH 8.0), 100 mM $MgCl_2$, 500 mM NaCl, and 500 mM KCl), 1 µl PvuI (6 units, BRL) and 39 µl $H_2O$. The reaction is incubated at 37°C for 1 hour followed by ethanol precipitation. The pellet is resuspended in 39 µl $H_2O$. To this is added 5 µl 10X HindIII buffer (500 mM Tris-HCl (pH 8.0), 100 mM $MgCl_2$, and 500 mM NaCl) and 1 µl HindIII (8 units, BRL). The desired ~4.8 kb PvuI/HindIII restriction fragment is separated and isolated by agarose gel electrophoresis in substantial accordance with the method of Example 2B. The purified fragment is then dissolved in about 100 µl of TE buffer and stored at 0°C

for future use.

B. Isolation of the ~2.1 kb PvuI/ SphI Fragment from Plasmid pOGO4

Plasmid pOGO4 can be isolated from E. coli K12 JM109/pOGO4 in substantial accordance with the teaching of Example 1. Approximately 5 μg of plasmid pOGO4 are cut to completion with the restriction enzymes PvuI and SphI as follows. Five μl (~5 μg) of plasmid pOGO4 are mixed with 5 μl 10X SphI buffer (500 mM Tris-HCl (pH 7.4), 60 mM $MgCl_2$, 500 mM KCl, and 500 mM NaCl), 39 μl $H_2O$ and 1 μl SphI (6 units, BRL). The reaction is incubated for 1 hour at 37°C, then ethanol precipitated. The pellet is resuspended in 39 μl $H_2O$. To this is added 5 μl 10X PvuI buffer (500 mM Tris-HCl (pH 8.0), 100 mM $MgCl_2$, 500 mM NaCl, and 500 mM KCl) and 1 μl PvuI (6 units, BRL). The reaction is incubated at 1 hour for 37°C. The desired ~2.1 kb PvuI/SphI restriction fragment, which contains the Aspergillus nidulans acyltransferase gene and regulatory elements, is separated and isolated by agarose gel electrophoresis in substantial accordance with the method of Example 2B. The purified fragment is then resuspended in about 100 μl of TE buffer and stored at 0°C for future use.

C. Construction of an SphI/ HindIII DNA linker

The desired linker has the following sequence:

```
5'      CGGATCCA      3'
        ||||||||
3' GTACGCCTAGGTTCGA 5' .
```

It can be conventionally synthesized by the modified phosphotriester method in substantial accordance with the teaching of Itakura et al., Science 198:1056 (1977) and Crea et al., Proc. Nat. Acad. Sci. USA 75:5765 (1978).

Example 4

Final Construction of Plasmid pOW1061

About 20 picomoles of the synthetic DNA linker created in Example 3C are ligated to ~0.5 μg of the ~2.1 kb PvuI/SphI fragment isolated from plasmid pOGO4. The ligation is performed in substantial accordance with the teaching of Example 2D. The resultant fragment is then digested with HindIII in substantial accordance with the teaching of Example 3A, and the resultant PvuI/HindIII fragment is conventionally separated and isolated by gel filtration. After the addition of 3M sodium acetate to a final concentration of 0.2M, the DNA is precipitated with 3 volumes of 100% ethanol. The desired ~2.1 kb PvuI/HindIII fragment is resuspended in 100 μl of TE buffer and stored at 0°C for future use.

The final construction of plasmid pOW1061 is achieved by ligation of the ~4.8 kb PvuI/HindIII fragment isolated from plasmid pRH5 to the ~2.1 kb PvuI/HindIII fragment of the previous paragraph. About 1 μg of the ~4.8 kb PvuI/HindIII fragment and ~0.5 μg of the ~2.1 kb PvuI/HindIII fragment are ligated, and the resultant plasmid is used to transform E. coli K12 JM109. The ligation and transformation are performed in substantial accordance with the teaching of Example 2D. Some of the resultant transformants, as shown by agarose gel electrophoresis and other tests, contain only the desired ~6.8 kb pOW1061 plasmid (as shown in Figure 8). Such a transformant, herein designated E. coli K12 JM109/pOW1061, is selected, plated on TY agar (TY broth + 15 g/liter agar) containing apramycin at 100 μg/ml and then cultured using conventional microbiological techniques. The resultant transformants are used to isolated plasmid pOW1061, as described in Example 1.

Example 5

Increased Levels of Acyltransferase Enzyme and Penicillin G production in Aspergillus nidulans by Gene Dosage

A. Transformation of Aspergillus with Plasmid pOW1061

Plasmid pOW1061 is an acyltransferase expression vector constructed for use in an Aspergillus host. The intracellular concentration of acyltransferase activity in Aspergillus nidulans can be increased by the presence of additional acyltransferase genes, as provided by plasmid pOW1061. Additionally, an Aspergillus transformant containing multiple acyltransferase genes may exhibit an increased yield of penicillin G, provided the concentration of substrate is not limiting. Such a scenario would best be accomplished by selecting an Aspergillus host strain that produces penicillin G and accumulates isopenicillin N.

Preparation of Aspergillus protoplasts and transformation of an Aspergillus nidulans strain such as ATCC 28901 using plasmid pOW1061 may be accomplished in substantial accordance with the procedure of Yelton et al., Proc. Natl. Acad. Sci. USA 81:1470 (1984) as described below.

Siliconized liter flasks containing 400 ml of minimal medium plus 4 mM filter-sterilized L-tryptophan are inoculated with 8 x $10^8$ conidia and shaken at room temperature for 18 hours. Minimal medium is (per liter) (pH to 6.5):

| | |
|---|---|
| 200 ml | stock salt solution |
| 50 ml | 20% glucose solution |
| 4 ml | 26% $MgSO_4 \cdot 7H_2O$ |

Stock salt solution is (per liter):

| | |
|---|---|
| $NaNO_3$ | 60 g |
| KCl | 5.2 g |
| $KH_2PO_4$ | 15.2 g |

Trace Element Solution 10 ml

Trace element solution is (per 100 ml):

| | |
|---|---|
| $FeSO_4 \cdot 7H_2O$ | 0.1 g |
| $ZnSO_4 \cdot 7H_2O$ | 0.88 g |
| $CuSO_4 \cdot 5H_2O$ | 0.04 g |
| $MnSO_4 \cdot 4H_2O$ | 0.015 g |
| $Na_2B_4O_7 \cdot 10H_2O$ | 0.01 g |
| $(NH_4)_3Mo_7O_2 \cdot 4H_2O$ | 0.005 g |

The mycelium was harvested by filtration through cheese cloth, washed with 0.6 M $MgSO_4$, and squeezed and blotted with paper towels to remove excess liquid. The cells are suspended in filter-sterilized osmotic medium (1.2 M $MgSO_4$/10 mM sodium phosphate, pH 5.8; 5 ml/g of mycelium) by vigorous mixing, transferred to a 250 ml flask, and placed on ice. Filter-sterilized solutions of β-glucuronidase (0.2 ml/g of mycelium; Sigma Chemical Co., P.O. Box 14508, St. Louis, MO 63178) and Novozyme 234 (20 mg/ml in osmotic medium; 1 ml/g of mycelium; Novo industri A/B Bagsvaerd, Denmark) are added, and the cells are incubated on ice for 5 minutes. A filter-sterilized solution of bovine serum albumin (12 mg/ml in osmotic medium; 0.5 ml/g of mycelium; Sigma) is added, and the cell suspension is shaken at 80 rpm at 30°C for 90 minutes. The suspension is transferred to a centrifuge tube, overlayed with 10 ml of ST buffer (0.6 M sorbitol (Sigma)/100 mM Tris-HCl (pH 7.0), and centrifuged in a swinging bucket rotor at 4000 x g at 4°C for 15 minutes. Protoplasts at the buffer interface are removed by using a bent Pasteur pipette and placed on ice. The remaining ST buffer is removed, the mycelial pellet is resuspended, fresh ST buffer is added as before, and protoplasts are again banded by centrifugation. The protoplasts are pooled, diluted with two volumes of STC buffer (1.2 M sorbitol 10 mM Tris-HCl (pH 7.5), 10 mM $CaCl_2$), and pelleted by centrifugation at 8000 x g at 4°C for 5 minutes. They are then washed twice by centrifugation with 10 ml of STC buffer and resuspended in 1/1000th of the original culture volume of STC buffer.

Plasmid pOW1061 DNA, dissolved in 25 µl of STC buffer, is mixed with 100 µl of protoplasts in a disposable plastic centrifuge tube and incubated at room temperature for 25 minutes. Then 0.2 ml of 60% polyethylene glycol 4000 in 10 mM Tris-HCl (pH 7.5) and 10 mM $CaCl_2$ is added, and the tube is agitated gently by hand. This is followed by a second addition of 0.2 ml and a third addition of 0.85 ml of the polyethylene glycol solution, with gentle mixing after each addition. The protoplasts are incubated for 20 minutes at room temperature and pelleted at 8000 x g at 4°C for 5 minutes. The supernatant is decanted, and droplets of solution adhering to the tube are removed with a cotton swab. The protoplasts are suspended in 2.5 ml of 0.5% yeast extract, 2.0% glucose, and 1.2 M sorbitol and incubated at 37°C on a rotary shaker at 150 rpm for 2 hours. They are then pelleted at 8000 x g at 4°C for 5 minutes, the supernatant is decanted, and droplets of medium adhering to the tube are removed with a cotton swab. The protoplasts are finally suspended in 0.15 ml of STC buffer and diluted appropriately in the same buffer.

After transformation, protoplasts are plated on non-selective medium (Trypticase Soy Agar, BBL Microbiology Systems, Becton Dickinson and Co., Cockeysville, MD 21030) and incubated overnight at 15°C to allow expression of the phleomycin resistance gene. An overlay of 4 ml soft agar (0.4% agar, Gibco Diagnostics, Madison, WI 53713) containing phleomycin is then added to the plates. The final plate concentration of phleomycin should be about 1 µg/ml. Incubation at 30°C thereafter yields transformants in about 4-7 days.

B. Analysis of Aspergillus Transformants and Detection of Penicillin G

Aspergillus transformants exhibiting stable phleomycin resistance can be subsequently picked and transferred to fresh non-selective Trypticase Soy Agar. After growth at 30°C for 7-10 days, cell extracts can be prepared according to the method of Dotzlaf, J.E. and Yeh, W.-K., J. Bacteriol. 169:1611 (1986), and assayed for penicillin G production as described by Neuss et al., J. Antibiot. 35:580 (1982).

Example 6

Increased Penicillin G Production in Penicillium chrysogenum by Gene Dosage Using the Aspergillus nidulans Acyltransferase Gene

Plasmid pOW1061 (constructed in Example 4) can also be used to increase acyltransferase enzyme level and penicillin G production in Penicillium chrysogenum. Penicillium chrysogenum has been shown to utilize Aspergillus regulatory elements and recognize intron splice sites in heterologous transformation experiments (Beri, R.J., and Turner, G., Curr. Genet. 11:639 (1987)). As in the case with an Aspergillus host, it is advantageous to select a Penicillium host that makes penicillin G and also accumulates isopenicillin N. A Penicillium transformant containing the cloned Aspergillus acyltransferase gene could produce higher levels of penicillin G than the parental strain, provided that the concentration of substrate is not limiting. Transformation of Penicillium chrysogenum can be performed as described below.

Example 7

Genetic Transformation of Penicillium

A. Penicillium chrysogenum Strains

A Penicillium strain for transformation is obtained from the American Type Culture Collection, Rockville, MD 20852, under the accession number ATCC 9480. Other Penicillium chrysogenum strains or any commercial strains derived from ATCC 9480 by mutation, selection, or genetic breeding for the purpose of improved production of penicillin G or penicillin V are also suitable for use in preparing transformants with the vectors and plasmids of the present invention.

B. Preparation of Uniform Inoculum for Cell Culture

To transform Penicillium chrysogenum cells efficiently, it is necessary to remove the cell walls to form stable protoplasts. In the preparation of such protoplasts it is advantageous to begin with a uniform inoculum. Otherwise, preparation of cells in culture is not reproducible and time is lost by attempts to prepare P. chrysogenum protoplasts from unsuitable or inadequate amounts of cells.

An ampoule of vegetative cells (~$10^9$ colony forming units in 1.0 ml of preservation menstruum: 5% lactose, 10% glycerol, and 0.1% Tween 80), either lyophilized or taken from liquid nitrogen storage and thawed at room temperature, are diluted in 1.0 ml of sterile saline. About 0.1 ml of this suspension is used to inoculate each of approximately 20 slants of sporulation medium: Lactose, 15.0 g/L; corn steep liquor, 2.5 g/L; peptone, 5.0 g/L; NaCl, 4.0 g/L; $MgSO_4 \cdot 7H_20$, 0.5 g/L; $KH_2PO_4$, 0.6 g/L; $FeCl_3 \cdot 6H_20$, 0.005 g/L; $CuS0_4 \cdot 5H_20$, 0.002 g/L; adjust to pH=7.0; agar, 30.0 g/L; and autoclave 20 minutes at 120 psi.

Each slant [15 cm x 2.5 cm] contains 25 ml of solidified medium. Inoculum, spread evenly over the surface of the agar slant, is allowed to grow at 25°C until a confluent lawn of mycelium is present and sporulated (1 week for most strains). The growth from 1 slant is suspended in 10 ml of sterile aqueous culture medium, and the suspension is transferred to 106 ml of aqueous culture medium. The flask containing the suspended cells is placed on a gyratory shaker and incubated at 25°C for 18 hours at 285 rpm with a 1 inch throw.

Aqueous culture medium was prepared as follows: 100 ml of solution A (Sucrose, 36 g/L; L-asparagine, 7.5 g/L; $KH_2PO_4$, 15 g/L; $K_2HPO_4$, 21 g/L; $NaSO_4$, 0.75 g/L, $MgSO_4 \cdot 7H_2O$, 0.18 g/L; $CaCl_2$, 0.06 g/L; salts solution, 1 ml/L; and natural pH) are dispensed into a 500 ml shake flask; the flask is covered with a commercial closure and autoclaved at 121°C for 20 minutes. Two ml of solution B (Glucose, 108 g/L) and 4 ml of solution C (Sucrose, 25 g/L; corn steep liquor (4% w/v nitrogen), 12.5 ml; ammonium acetate, 5.5 g/L; $CaCO_3$, 5 g/L; pH adjusted to 6.5 with KOH; and autoclaved at 121°C for 20 minutes) are then added to solution A to prepare the aqueous culture medium.

C. Preparation of Penicillium protoplasts

Cells from a 24 hour culture are harvested by suction filtration (Whatman #1 paper in a Buchner funnel) and suspended in buffer (0.01 M Tris; 0.01 M $MgSO_4$; 0.01 M dithiothreitol; 1.00 M KCl; and pH=7.0 with HCl). Sufficient buffer is added to obtain a final cell concentration of 1 g of cell mass per 50 ml of buffer. The cell suspension is placed on a gyratory water bath shaker in a 250 ml shake flask and incubated at 29-30°C for 10 minutes at 140 rpm with a 1 inch throw. Dithiothreitol-treated cells are collected by centrifugation and then resuspended in 50 ml of enzyme solution (10 mg/ml Novozym 234, Novo industri A/B Bagsvaerd, Denmark; 0.01 M Tris; 0.01 M $MgSO_4$; 0.01 M dithiothreitol; 1.00 M KCl; and pH=5.8 with HCl) in a 250 ml shake flask. This cell suspension is placed on a gyratory water-bath shaker and incubated at 29-30°C for 15 minutes at 140 rpm with a 1 inch throw. Enzyme-treated cells are centrifuged at 1240Xg for 6 min, and the resulting pellet is resuspended in buffer (0.01 M Tris(hydroxymethyl)aminomethane hydrochloride; 0.01 M $MgSO_4$; 1.00 M KCl; and pH=7.0 with HCl). The suspension is first centrifuged at 950Xg for 6 minutes. The resulting pellet is resuspended in the same buffer, and the suspension is centrifuged at 700Xg for 6 minutes. The resulting pellet is resuspended in 5 ml of the same buffer. This suspension contains primarily large protoplasts and osmotically fragile cells that retain some cell wall structure. Compared to the small protoplasts removed by the above procedure, the percentage of protoplasts able to regenerate cell walls and percentage of viable osmotically stable cells is higher for the large protoplasts and osmotically fragile cells in the final suspension. The suspension of cells is diluted with buffer to a concentration of ~2 x $10^8$ cells/ml.

D. Transformation Procedure

For each transforming plasmid, an ~0.1 ml suspension of osmotically fragile Penicillium chrysogenum cells (approximately 2 x $10^7$ cells) is supplemented with 10 µl of 50 mM $CaCl_2$, 25 µg of plasmid DNA in 5-15 µl of TE buffer, and 0.9 ml of a solution of freshly dissolved polyethylene glycol 4000 (Baker, 40% weight/volume in osmotically stabilized buffer). The mixture is vortexed, allowed to stand for 10 minutes at room temperature, centrifuged at 700Xg for 2 minutes, and vortexed again. The protoplasts may be plated and the transformants analyzed as with the Aspergillus transformation described in Example 5.

Example 8

Increased Penicillin G Production in Penicillium chrysogenum Using the Aspergillus nidulans Acyltransferase Gene with the Penicillium chrysogenum Isopenicillin N Synthetase Promoter

While plasmid pOW1061 can be used in Penicillium to increase acyltransferase enzyme level and penicillin G production, it would be advantageous to construct a plasmid more specifically designed to drive higher levels of expression of the Aspergillus nidulans acyltransferase in Penicillium. Such a plasmid, described in this example as pOW1062 (See Figure 9), employs the Penicillium chrysogenum isopenicillin N synthetase promoter to achieve higher levels of expression of the acyltransferase gene from Aspergillus nidulans in Penicillium.

Construction of Plasmid pOW1062

A. Isolation of the Penicillium chrysogenum IPNS Promoter

The ~0.9 kb HindIII/NcoI fragment containing the DNA immediately upstream of the Penicillium chrysogenum IPNS gene is isolated from plasmid pLC2 in substantial accordance with the teaching of Example 2B.

B. Construction of the NcoI/ KpnI DNA Linker

The sequence of the desired linker is as follows:

```
5' CATGCTTCACGTAACTTGCCAAGGTAC 3'
       |||||||||||||||||||
       GAAGTGCATTGAACGGTTC
```

The linker is synthesized by the modified phosphotriester method in substantial accordance with the teaching of Example 3C.

C. Construction of an ~0.9 kb HindIII/KpnI DNA Fragment

About 20 picomoles of the DNA linker of Example 8B and ~0.5 µg of the ~0.9 kb HindIII/NcoI fragment isolated from plasmid pLC2 in Example 8A are ligated in substantial accordance with Example 2D. The resultant fragment is

then digested with 6 units KpnI in 1X KpnI buffer (20 mM Tris-HCl (pH 7.4), 5 mM $MgCl_2$, and 50 mM KCl) for 1 hour at 37°C, and the resultant HindIII/KpnI fragment is conventionally isolated by gel filtration followed by ethanol precipitation.

D. Isolation of the ~2.2 kb KpnI/ HindIII Fragment from Plasmid pOGO4

Plasmid pOGO4 is isolated from E. coli K12 JM109/pOGO4, available from the NRRL under accession number NRRL B-18171, in accordance with the procedure of Example 1. Approximately 5 µg of plasmid pOGO4 are cut to completion with the restriction enzyme KpnI as in Part C, above, followed by digestion with 8 units HindIII in 1X HindIII buffer (50 mM Tris-HCl (pH 8.0), 10 mM $MgCl_2$, and 50 mM NaCl) for 1 hour at 37°C, and the desired ~2.2 kb KpnI/HindIII restriction fragment is isolated by agarose gel electrophoresis in accordance with Example 2B. In the event that the desired ~2.2 kb KpnI/HindIII fragment is not easily separable from the other digestion products, an additional digestion with EcoRV is recommended. The purified ~2.2 kb KpnI/HindIII fragment is then dissolved in about 100 µl of TE buffer and stored at 0°C for future use.

E. Construction of the ~5 kb HindIII Fragment of Plasmid pRH5

Plasmid pRH5 is isolated from E. coli K12 JM109/pRH5 (constructed in Example 2) as described in Example 1. Approximately 5 µg of plasmid pRH5 are digested with the restriction enzyme HindIII as in Example 8D, and the resultant ~5 kb HindIII restriction fragment is isolated by agarose gel electrophoresis as in Example 2B. The purified fragment is then dissolved in about 100 µl of TE buffer and stored at 0°C for future use.

F. Construction of plasmid pOW1062

About 1 µg of the ~5 kb HindIII fragment from plasmid pRH5, about 0.5 µg of ~0.9 kb HindIII/KpnI fragment of Example 8C, and about 0.5 µg of ~2.2 kb KpnI/HindIII fragment of plasmid pOGO4 are ligated, and the resultant plasmid is used to transform E. coli K12 JM109 to resistance to apramycin at 100 µg/ml. The ligation and transformation are performed in substantial accordance with the teaching of Example 2D. Some of the resultant transformants, as conventionally shown by restriction mapping and agarose gel electrophoresis and other tests, contain only the ~8 kb plasmid, as shown in Figure 9. Such a transformant, herein designated E. coli K12 JM109/pOW1062, is selected, plated on TY agar containing 100 µg/ml apramycin and then cultured using conventional microbiological techniques. The resultant transformants are used to isolate plasmid pOW1062, as described in Example 1.

Example 9

Gene Disruption of the Acyltransferase Gene in Aspergillus nidulans

An Aspergillus nidulans strain which is incapable of penicillin G synthesis, instead accumulating isopenicillin N is best made from a penicillin G-producing Aspergillus strain known to contain only one copy of the acyltransferase gene. The desired AT-minus strain is constructed by interrupting the genomic acyltransferase gene with a non-functional acyltransferase gene in an homologous single crossover event. An appropriate non-functional gene can be constructed by removing segments from both the 5′ and 3′ ends of the gene.

I. Construction of Plasmid pOW1063

A. Isolation of the ~5 kb Blunt-ended Fragment of Plasmid pRH5

Plasmid pRH5 (constructed in Example 2) is isolated from E. coli K12 JM109/pRH5 as described in Example 1. Approximately 5 µg of plasmid pRH5 are cut to completion with 8 units EcoRI in 1X EcoRI buffer (50 mM Tris-HCl (pH 8.0), 10 mM $MgCl_2$, and 0.1 M NaCl) for 1 hour at 37°C, and the resultant ~5 kb EcoRI fragment is then treated with E. coli Klenow fragment as follows in order to blunt the DNA termini. Five µl (~5 µg) of the EcoRI-digested plasmid pRH5 is mixed with 1 µl of a solution containing 2 mM of each dNTP, 2.5 µl 10X buffer (0.5 M Tris-HCl (pH 7.2), 0.1 M $MgSO_4$, and 1 mM DTT) and 16.5 µl $H_2O$. Then 2 units of Klenow fragment is added and the mixture incubated for 15 minutes at 22°C. The enzyme is then heat-inactivated by treating for 5 minutes at 70°C. The resultant ~5 kb blunt-ended DNA fragment is conventionally isolated by gel electrophoresis as in Example 2B, and ethanol precipitated. The desired ~5 kb blunt-ended fragment is dissolved in 100 µl TE buffer and stored at 0°C for future use.

B. Isolation of the ~0.8 kb Blunt-ended Fragment from Plasmid pOGO4

Plasmid pOGO4 (NRRL B-18171) is isolated from E. coli K12 JM109/pOGO4 as described in Example 1. Approximately 5 µg of plasmid pOGO4 are cut to completion with the restriction enzyme NcoI as described in Example 9IA except that NcoI is used instead of EcoRI, and the DNA termini are filled in as described in Example 9IA. The ~0.8 kb blunt-ended fragment containing a truncated AT gene is separated and isolated by agarose gel electrophoresis as in Example 2B. The purified ~0.8 kb fragment is then dissolved in about 100 µl of TE buffer and stored at 0°C for future use.

C. Construction of Plasmid pOW1063

About 1 µg of the ~5 kb blunt-ended fragment constructed in Example 9A and 0.5 µg of the ~0.8 kb blunt-ended fragment constructed in Example 9B are ligated, and the resultant plasmid is used to transform E. coli K12 JM109. The ligation and transformation are performed in substantial accordance with Example 2D. Some of the transformants, as conventionally shown by agarose gel electrophoresis and other tests, contain only the desired ~5.7 kb plasmid (Figure 10). Such a transformant, herein designated E. coli K12 JM109/pOW1063, is selected, plated on TY agar containing apramycin at 100 µg/ml and then cultured using conventional microbiological techniques. The resultant transformants are used to isolate plasmid pOW1063, as described in Example 1.

II. Transformation of Aspergillus nidulans with Plasmid pOW1063

Preparation of Aspergillus protoplasts and transformation of an Aspergillus nidulans strain such as ATCC 28901 using plasmid pOW1063 may be accomplished in substantial accordance with the procedure of Example 5.

III. Analysis and Detection of Acyltransferase-minus Aspergillus Transformants

Aspergillus transformants are isolated and grown as previously described in Example 5B, and cell extracts were prepared and assayed for penicillin G production. Some of the transformants will be unable to synthesize penicillin G due to a homologous recombination event involving the interruption of the genomic acyltransferase gene. Such transformants contain two incomplete, non-functional AT genes. One AT lacks 3' coding sequence while the other lacks a portion of the 5' coding region. The process is shown in the diagram below.

Transform Aspergillus with Plasmid pOW 1063

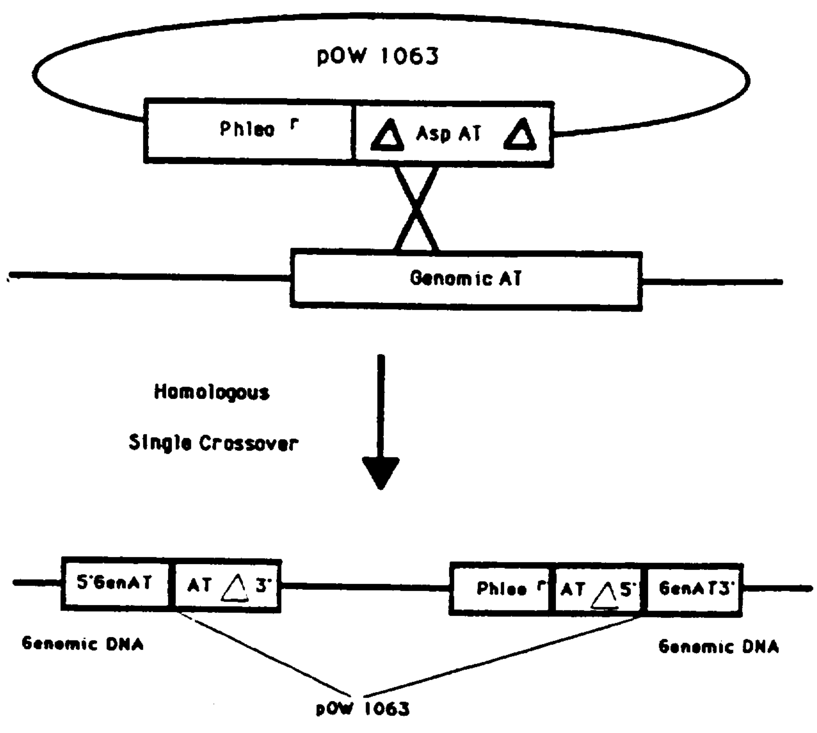

The desired transformant accumulates isopenicillin N as the end product in its β-lactam biosynthetic pathway. Conventional verification of the transformant includes restriction enzyme analysis of the genomic DNA, as well as other tests. Penicillin G biosynthetic capability is restored to the desired Aspergillus transformant by transforming it with plasmid pOW1061 (refer to Example 3).

Example 10

Gene Displacement of the Acyltransferase Gene in Aspergillus nidulans

Another method of generating an Aspergillus strain incapable of producing penicillin G is by gene displacement. The genomic acyltransferase gene is displaced by a nonfunctional acyltransferase gene by a protocol favoring a homologous double-crossover event. The desired non-functional gene is constructed by interrupting the plasmid-borne acyltransferase gene with a resistance marker. In this case, the phleomycin resistance gene with the Penicillium IPNS promoter is used.

I. Construction of Intermediate Plasmid pOW1064

A. Isolation of the ~2.7 kb HindIII/ PvuI Fragment of Plasmid pKC787

Approximately 5 µg of plasmid pKC787 (Example 2C) are cut to completion with the restriction enzymes PvuI and HindIII in substantial accordance with the teaching of Example 3A, and the desired ~2.7 kb PvuI/HindIII fragment is isolated by agarose gel electrophoresis as in Example 2B. The purified fragment is then dissolved in about 100 µl of TE buffer and stored at 0°C for future use.

B. Isolation of the ~2.8 kb HindIII/ PvuI Fragment of Plasmid pOGO4

Plasmid pOGO4 (NRRL B-18171) is obtained from E. coli K12 JM109/pOGO4 as described in Example 1. About 5 µg of plasmid pOGO4 are digested with HindIII and PvuI as in Example 3A, and the desired ~2.8 kb HindIII/PvuI fragment containing the AT gene is then isolated by agarose gel electrophoresis as in Example 2B. The purified fragment is dissolved in about 100 µl of TE buffer and stored at 0°C for future use.

C. Construction of Plasmid pOW1064

About 1 µg of the ~2.7 kb HindIII/PvuI fragment from plasmid pKC787 and about 0.5 µg of plasmid pOGO4 ~2.8 kb HindIII/PvuI fragment are ligated, and the resultant ligation reaction is used to transform E. coli K12 JM109/pOW1064 to resistance to 100 µg/ml aparamycin. The ligation and transformation is performed in substantial accordance with the procedure of Example 2D. Some of the transformants, as shown by agarose gel electrophoresis and other tests, contain only the desired ~5.5 kb plasmid (refer to Figure 11 of the accompanying drawings). Such a transformant, herein designated E. coli K12 JM109/pOW1064, is selected, plated on TY agar containing 100 µg/ml apramycin and then cultured using conventional microbiological techniques. The resultant transformants are used to isolate plasmid pOW1064, as described in Example 1.

II. Construction of Plasmid pOW1065

A. Isolation of the ~4.7 kb NcoI Fragment from Plasmid pOW1064

Plasmid pOW1064 (as constructed above) is isolated from E. coli K12 JM109/pOW1064, as described in Example 1. Approximately 5 µg of plasmid pOW1064 is digested with NcoI and the termini of the fragments are made blunt with Klenow enzyme in substantial accordance with the teaching of Example 9IB. The resultant ~4.7 kb fragment is conventionally isolated by agarose gel electrophoresis. The resultant ~4.7 kb fragment is dissolved in about 100 µl of TE buffer and stored at 0°C for future use.

B. Isolation of the ~2.1 kb Blunt-ended Fragment Containing the Phleomycin Gene and Penicillium IPNS Promoter

Plasmid pRH5 (constructed in Example 2) is isolated from E. coli as described in Example 1. About 5 µg of plasmid pRH5 is digested with EcoRI in substantial accordance with Example 9IA and HindIII in substantial accordance with Example 8D, and the resultant fragments are filled in at their termini with Klenow enzyme in substantial accordance with Example 9IB. One of the resultant fragments is an ~2.1 kb blunt-ended restriction fragment which is conventionally

isolated by agarose gel electrophoresis. The desired ~2.1 kb blunt-ended fragment is dissolved in 100 µl of TE buffer and stored at 0°C for future use.

C. Construction of Plasmid pOW1065

About 1 µg of the ~4.7 kb blunt-ended fragment of Example 10-IIA and about 0.5 µg of the ~2.1 kb blunt-ended fragment of Example 10-IIB are ligated, and the resultant plasmid is used to transform E. coli K12 JM109 to resistance to 100 µg/ml apramycin. The ligation and transformation are performed in substantial accordance with Example 2D. Some of the transformants, as conventionally shown by agarose gel electrophoresis and other tests, contain only the ~6.8 kb plasmid (as shown in Figure 12 of the accompanying drawings). Such a transformant, herein designated E. coli K12 JM109/pOW1065, is selected, plated on TY agar containing 100 µg/ml apramycin and then cultured using conventional microbiological techniques. The resultant transformants are used to isolate plasmid pOW1065, as described in Example 1.

III. Transformation of Aspergillus nidulanswith Plasmid pOW1065

Preparation of Aspergillus protoplasts and transformation of an Aspergillus nidulans strain such as ATCC 28901 using plasmid pOW1065 is accomplished as in Example 5. Linearizing the plasmid before transformation increases the probability of obtaining a transformant containing the desired homologous double crossover. This is accomplished by HindIII digestion of plasmid pOW1065 prior to transformation.

IV. Analysis and Detection of Acyltransferase-minus Aspergillus Transformants

Aspergillus transformants are isolated and grown as previously described in Example 5B, cell extracts prepared and assayed for penicillin G production. Some of the transformants are unable to synthesize penicillin G due to an homologous recombination event involving a double crossover. In such an event, the functional genomic acyltransferase gene will be lost from the genome, and replaced by the mutant nonfunctional acyltransferase gene carried on plasmid pOW1065 as shown below.

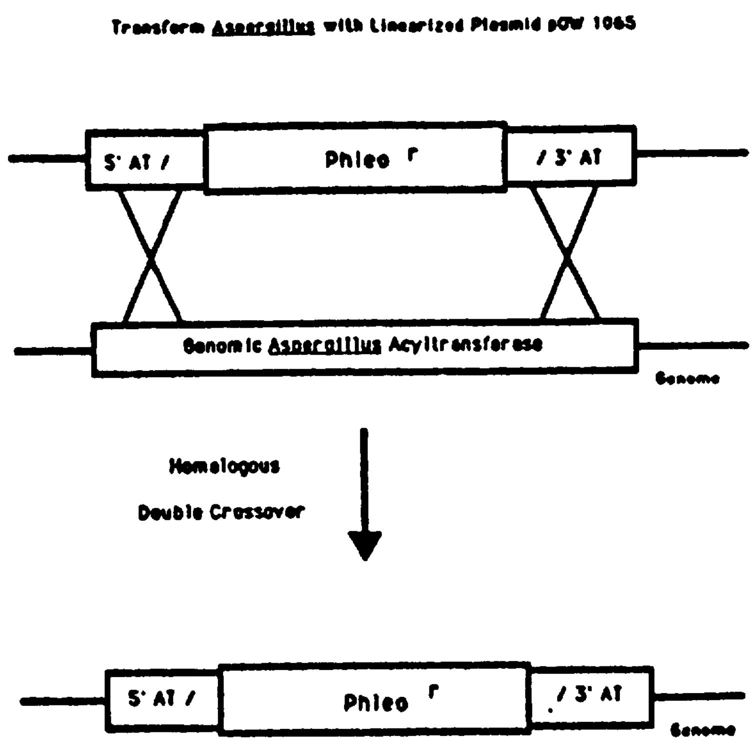


The desired transformants will additionally accumulate isopenicillin N. Further verification of such a transformant may include restriction enzyme analysis of the Aspergillus genomic DNA coupled with Southern hybridization analysis. Transformation of the AT-minus strain with plasmid pOW1061 (see Example 3) will restore the penicillin G biosynthetic capability of the strain, thus providing additional means for determining that the desired double crossover event has occurred.

Example 11

Production of Cephalosporins in Aspergillus AT-minus Strains

The Aspergillus AT-minus strains created in Examples 9 and 10 are exploited to produce useful cephalosporins in Aspergillus by the introduction into the strains of genes encoding deacetoxycephalosporin C synthetase (expandase) and isopenicillin N epimerase (epimerase). Any such genes should be suitable as long as they are under the control of transcriptional and translational control regions that function in the host organism.

An especially preferred set of expandase and epimerase genes is derived from Streptomyces clavuligerus. The expandase gene of S. clavuligerus can be derived from plasmid pOW380, available from the NRRL in the E. coli K12 strain RR1ΔM15 under accession number NRRL B-18264. The epimerase gene of S. clavuligerus is available on plasmid pOW390 in E. coli K12 RR1ΔM15 from the NRRL under accession number NRRL B-18431. The genes must then be put under the control of any of the commonly known promoters that function in Aspergillus, such as the Penicillium isopenicillin N synthetase promoter described in U.S. Patent Application No. 06/801,523 (European Patent Publication Number 0 225 128) and available on plasmid pLC2 (ATCC 53334).

Vectors comprising the expandase and epimerase genes are then transformed into the Aspergillus AT-minus strains by the method of Example 5. The transformants are picked and grown at 30°C for 7-10 days and conventionally assayed for cephalosporin production.

Example 12

Production of Cephalosporins in Penicillin G-Producing Fungi Using Acyltransferase Anti-Sense RNA

I. Construction of Plasmid pPS95

A. Preparation of a DNA Fragment Containing the P. chrysogenum Acyltransferase Promoter Bounded by HindIII and NcoI Restriction Sites

The Penicillium chrysogenum isopenicillin N synthetase promoter, available on plasmid pLC2 (ATCC 53334) is functional in both Aspergillus and Penicillium. Although this Example describes a procedure using the Aspergillus acyltransferase gene in Aspergillus, an analogous procedure can be carried out in Penicillium with the Penicillium acyltransferase gene. One need merely substitute the Penicillium AT coding region for that of Aspergillus in this Example. The Penicillium AT gene can be isolated from a library of genomic DNA from any publicly available Penicillium strain using the AT gene of the present invention as a hybridization probe.

The polymerase chain reaction (PCR) is used to amplify a double-stranded DNA fragment from plasmid pLC2, isolated as in Example 1. The fragment contains the 5′ regulatory region of the acyltransferase gene. The reaction (apparatus and reagents available from (Perkin Elmer Cetus, 1400 53rd St., Emeryville, CA 94608) is performed using the single-stranded primers depicted below. The reaction components are 200 μM each dNTP, 1 μM each primer, 1 ng template, and 2.5 units Amplitaq™ DNA polymerase in 1X buffer in a total of 100 μl. The reaction is incubated for 25 cycles of 1 minute at 94°C, 1 minute at 50-60°C, and 1 minute at 72°C. After the final cycle, incubation at 72°C should be continued for seven minutes to complete the polymerization of all strands. The DNA primers may be manually synthesized by the methods of Itakura et al. (1977) Science 198:1056; Crea et al. (1978) Proc. Natl. Acad. Sci. USA 75:5765; Hsuing et al. (1983) Nuc. Acid Res. 11:3227; or Narang et al. (1980) Methods in Enzymology 68:90. The primers may also be synthesized using automated DNA synthesizers, such as the ABS (Applied Biosystems, 850 Lincoln Centre Drive, Foster City, CA 94404) 380A DNA synthesizer. The first primer incorporates a HindIII site into the fragment. The sequence of the primer is:

HindIII

5'-AGCCAAGCTTTCAGGCAACCT

The second primer incorporates an NcoI site into the fragment and initiates priming 20 base pairs downstream from the translation initiation site.

NcoI

5'-CCAGCCATGGTTGCTGCGGGTCGGAAGATGG

The product of the PCR reaction is a DNA fragment 488 base pairs in length. A HindIII restriction site is located 4 base pairs from the 5′ end. An NcoI restriction site is located 4 base pairs from the 3′ end. The amplified DNA is extracted twice with phenol, twice with chloroform, ethanol precipitated, and resuspended in water. The fragment is then cut to completion with the restriction enzymes NcoI in substantial accordance with Example 9IA except that NcoI is used instead of EcoRI and HindIII in substantial accordance with Example 8D, phenol extracted, chloroform extracted, ethanol precipitated and resuspended in 25 µl water. The fragment is now about 478 base pairs long.

B. Preparation of a DNA Fragment Containing the Aspergillus nidulans Acyltransferase Open Reading Frame Bounded by HindIII and NcoI Restriction Sites

As in part A, supra, the PCR reaction is used to generate a quantity of a DNA fragment containing the acyltransferase gene bounded by a HindIII restriction site at the 5′ end and NcoI restriction site at the 3′ end. The template for the PCR reaction is plasmid pOGO4, isolated as described in Example 1. The primers for the reaction are illustrated below:

HindIII

5'-CAGCAAGCTTCTTCACGTAACTTGCCAAGG-3'

This primer initiates priming 20 base pairs from the 5′ end of the AT open reading frame.

The second primer initiates priming 20 base pairs from near the 3′ end of the AT open reading frame:

NcoI

5'-TTCGCCATGGAATGTTGGCTTGGATCGCGG-3'

The amplified DNA fragment is phenol extracted twice, chloroform extracted twice, ethanol precipitated, resuspended in $H_2O$, cut with HindIII and NcoI, extracted with phenol followed by chloroform, ethanol precipitated and resuspended in water. The DNA fragment produced is 1290 base pairs in length. After restriction enzyme cleavage it is about 1280 base pairs long.

C. Preparation of a DNA Fragment Containing the P. chrysogenum Acyltransferase Promoter Juxtaposed to the A. nidulans Acyltransferase Gene in the Anti-sense Orientation

Equimolar amounts of the DNA fragment produced in Part A and the DNA fragment produced in Part B are mixed and ligated in accordance with Example 2D. The ligase is inactivated by extraction with phenol, then chloroform. The DNA is then ethanol precipitated and resuspended in $H_2O$. The ligated material is then cut with restriction enzyme HindIII, which is then inactivated as above. The DNA fragments generated are resuspended in $H_2O$.

The desired product can be diagrammed in this way:

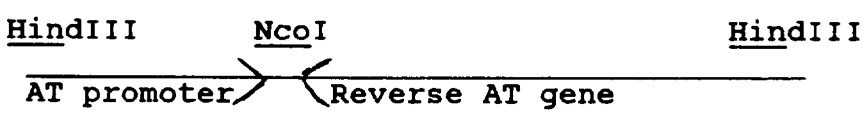


D. Preparation of HindIII-Cut pRH5 Plasmid DNA

Plasmid pRH5 (from Example 2) is cut to completion with restriction enzyme HindIII. The reaction mixture is phenol, then chloroform extracted, ethanol precipitated and resuspended in $H_2O$.

E. Final Construction of Plasmid pPS95

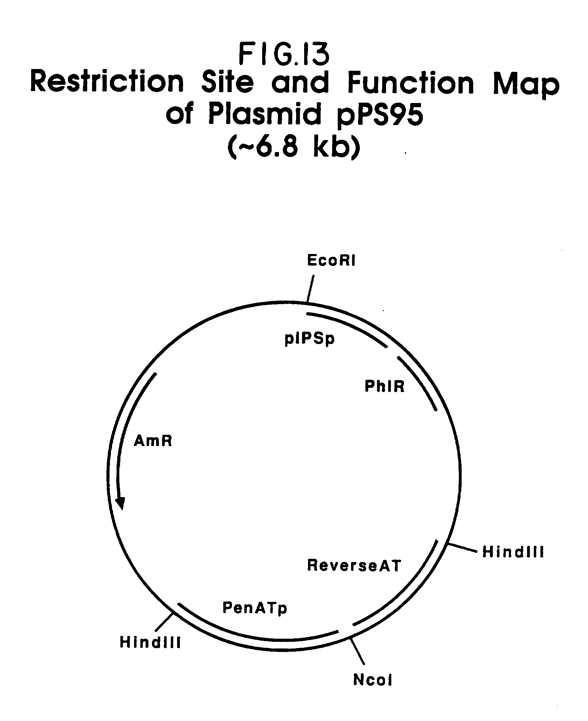

The DNA fragments produced in Part C are ligated to the HindIII-cut plasmid pRH5 DNA prepared in Part D to form the desired plasmid pPS95. The ligation mixture comprising the desired plasmid pPS95 is transformed into competent E. coli K12 JM109 cells. The ligation and transformation are performed in substantial accordance with Example 2D. Aliquots of the transformation reaction are plated on L-agar containing 100 µg/ml apramycin. Apramycin-resistant transformants were screened for an insert in pRH5 by gel electrophoresis. The HindIII fragment in pRH5 will be in one of two orientations. Plasmids in which the fragment is in the desired orientation are designated pPS95 and are conventionally identified by restriction enzyme analysis. A structure and function map of plasmid pPS95 is presented in Figure 13.

II. Production of Cephalosporins in Aspergillus Strains Comprising Plasmid pPS95

The Aspergillus strains comprising the AT-anti-sense plasmid pPS95 is used to produce cephalosporins as described in Example 11.

## Claims

### Claims for the following Contracting States : AT, BE, CH, DE, DK, FR, GB, GR, IT, LI, NL, SE

1. A DNA compound that comprises an isolated DNA sequence encoding isopenicillin N:acyl CoA acyltransferase activity of Aspergillus nidulans, said isolated DNA sequence encoding:

**MetLeuHisValThrCysGlnGlyThrProSerGluIleGlyTyrHisHisGlySer
AlaAlaLysGlyGluIleAlaLysAlaIleAspPheAlaThrGlyLeuIleHisGly
LysThrLysLysThrGlnAlaGluLeuGluGlnLeuLeuArgGluLeuGluGlnVal
MetLysGlnArgTrpProArgTyrTyrGluGluIleCysGlyIleAlaLysGlyAla
GluArgGluValSerGluIleValMetLeuAsnThrArgThrGluPheAlaTyrGly
LeuValGluAlaArgAspGlyCysThrThrValTyrCysLysThrProAsnGlyAla
LeuGlnGlyGlnAsnTrpAspPhePheThrAlaThrLysGluAsnLeuIleGlnLeu
ThrIleCysGlnProGlyLeuProThrIleLysMetIleThrGluAlaGlyIleIle
GlyLysValGlyPheAsnSerAlaGlyValAlaValAsnTyrAsnAlaLeuHisLeu
HisGlyLeuArgProThrGlyLeuProSerHisLeuAlaLeuArgMetAlaLeuGlu
SerThrSerProSerGluAlaTyrGluLysIleValSerGlnGlyGlyMetAlaAla
SerAlaPheIleMetValGlyAsnAlaHisGluAlaTyrGlyLeuGluPheSerPro
IleSerLeuCysLysGlnValAlaAspThrAsnGlyArgIleValHisThrAsnHis
CysLeuLeuAsnHisGlyProSerAlaGlnGluLeuAsnProLeuProAspSerTrp
SerArgHisGlyArgMetGluHisLeuLeuSerGlyPheAspGlyThrLysGluAla
PheAlaLysLeuTrpGluAspGluAspAsnTyrProLeuSerIleCysArgAlaTyr
LysGluGlyLysSerArgGlySerThrLeuPheAsnIleValPheAspHisValGly
ArgLysAlaThrValArgLeuGlyArgProAsnAsnProAspGluThrPheValMet
ThrPheSerAsnLeuAspThrLysSerAlaIleGlnAlaAsnIle**

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a

methionine residue, Phe is a phenylalanine residue, Pro is a proline residue, Ser is a serine residue, Thr is a threonine residue, Trp is a tryptophan residue, Tyr is a tyrosine residue, and Val is a valine residue.

**2.** The DNA compound of Claim 1, wherein the coding strand comprises the isolated DNA sequence:

```
ATG CTT CAC GTA ACT TGC CAA GGT ACC CCC TCC GAA GTA AGT
CCA TAC CCG AAT GTA TAT TTG CCC ATA TTT AAC GCC TAT ACT
TCC AGA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC
TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG
```

```
GGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA
```

wherein A is a deoxyadenyl residue, G is a deoxyguanyl residue, C is a deoxycytidyl residue, and T is a thymidyl residue.

**3.** The ~2.1 kb PvuI-SphI restriction fragment of plasmid pOGO4 (obtainable from NRRL B-18171, and shown in Figure 3).

**4.** A recombinant DNA vector that comprises the isolated DNA sequence of Claim 1.

**5.** The recombinant DNA vector of Claim 4 that is pOW1061 (obtainable as described in Example 4, and shown in Figure 8) or pOW1062 (obtainable as described in Example 8, and shown in Figure 9).

**6.** A recombinant DNA vector of Claim 4 that further comprises a promoter and translational activating sequence positioned to drive expression of said acyltransferase activity-encoding DNA.

**7.** The recombinant DNA expression vector of Claim 6, wherein said promoter and translational activating sequence function in E. coli, Aspergillus, Penicillium or Cephalosporium.

**8.** The recombinant DNA expression vector of Claim 7, wherein said promoter is the E. coli λpL promoter.

**9.** The recombinant DNA expression vector of Claim 8, wherein said promoter is the promoter of the Penicillium chrysogenum IPNS gene.

**10.** The recombinant DNA expression vector of Claim 8, wherein said promoter and translational activating sequence function in Cephalosporium.

**11.** The recombinant DNA vector of Claim 10, wherein said promoter is the promoter of the Cephalosporium acremonium IPNS gene.

**12.** A method for constructing a recombinant host cell capable of expressing acyltransferase activity, said method comprising:
transforming said host cell with a recombinant DNA expression vector that comprises:

(a) a promoter and translational activating sequence that functions in said host cell; and
(b) said DNA sequence of Claim 1 positioned for expression from said promoter and translational activating sequence.

**13.** A method for expressing acyltransferase activity in a recombinant host cell, said method comprising:
culturing said transformed host cell of Claim 12 under conditions suitable for gene expression.

**14.** The method of Claim 13, wherein said recombinant host cell is E. coli, Penicillium or Aspergillus.

**15.** A recombinant host cell transformed with a recombinant DNA vector of Claim 4, wherein said transformed host cell is E. coli K12, Penicillium, or Aspergillus.

**16.** The DNA compound of Claim 1 wherein the coding strand comprises the isolated DNA sequence:

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT
CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

wherein A is a deoxyadenyl residue, G is a deoxyguanyl residue, C is a deoxycytidyl residue, and T is a thymidyl residue.

**Claims for the following Contracting State : ES**

1. A method for constructing a recombinant host cell capable of expressing acyltransferase activity, said method comprising:
   transforming said host cell with a recombinant DNA expression vector that comprises:

   (a) a promoter and translational activating sequence that functions in said host cell; and
   (b) a DNA sequence positioned for expresssion from said promoter and translational activating sequence, said DNA sequence encoding:

MetLeuHisValThrCysGlnGlyThrProSerGluIleGlyTyrHisHisGlySer
AlaAlaLysGlyGluIleAlaLysAlaIleAspPheAlaThrGlyLeuIleHisGly
LysThrLysLysThrGlnAlaGluLeuGluGlnLeuLeuArgGluLeuGluGlnVal
MetLysGlnArgTrpProArgTyrTyrGluGluIleCysGlyIleAlaLysGlyAla
GluArgGluValSerGluIleValMetLeuAsnThrArgThrGluPheAlaTyrGly
LeuValGluAlaArgAspGlyCysThrThrValTyrCysLysThrProAsnGlyAla
LeuGlnGlyGlnAsnTrpAspPhePheThrAlaThrLysGluAsnLeuIleGlnLeu
ThrIleCysGlnProGlyLeuProThrIleLysMetIleThrGluAlaGlyIleIle
GlyLysValGlyPheAsnSerAlaGlyValAlaValAsnTyrAsnAlaLeuHisLeu
HisGlyLeuArgProThrGlyLeuProSerHisLeuAlaLeuArgMetAlaLeuGlu
SerThrSerProSerGluAlaTyrGluLysIleValSerGlnGlyGlyMetAlaAla
SerAlaPheIleMetValGlyAsnAlaHisGluAlaTyrGlyLeuGluPheSerPro
IleSerLeuCysLysGlnValAlaAspThrAsnGlyArgIleValHisThrAsnHis
CysLeuLeuAsnHisGlyProSerAlaGlnGluLeuAsnProLeuProAspSerTrp
SerArgHisGlyArgMetGluHisLeuLeuSerGlyPheAspGlyThrLysGluAla
PheAlaLysLeuTrpGluAspGluAspAsnTyrProLeuSerIleCysArgAlaTyr
LysGluGlyLysSerArgGlySerThrLeuPheAsnIleValPheAspHisValGly
ArgLysAlaThrValArgLeuGlyArgProAsnAsnProAspGluThrPheValMet
ThrPheSerAsnLeuAspThrLysSerAlaIleGlnAlaAsnIle

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

**2.** A method for expressing acyltransferase activity in a recombinant host cell, said method comprising:
culturing said transformed host cell of Claim 1 under conditions suitable for gene expression.

**3.** The method of Claim 2, wherein said recombinant host cell is E. coli, Penicillium or Aspergillus.

**4.** A method according to any of Claims 1 to 3, wherein the coding strand comprises the isolated DNA sequence:

ATG CTT CAC GTA ACT TGC CAA GGT ACC CCC TCC GAA GTA AGT
CCA TAC CCG AAT GTA TAT TTG CCC ATA TTT AAC GCC TAT ACT
TCC AGA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC
TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG
GGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA

wherein A is a deoxyadenyl residue, G is a deoxyguanyl residue, C is a deoxycytidyl residue, and T is a thymidyl residue.

5. A method according to any of Claims 1 to 3, wherein the coding strand comprises the isolated DNA sequence:

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT
CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

wherein A is a deoxyadenyl residue, G is a deoxyguanyl residue, C is a deoxycytidyl residue, and T is a thymidyl residue.

## Patentansprüche

### Patentansprüche für folgende Vertragsstaaten : AT, BE, CH, DE, DK, FR, GB, GR, IT, LI, NL, SE

1. DNA Verbindung, gekennzeichnet durch eine isolierte DNA Sequenz, die Isopenicillin N:Acyl CoA Acyltransferase-Aktivität von Aspergillus nidulans kodiert, wobei diese isolierte DNA Sequenz kodiert für

**MetLeuHisValThrCysGlnGlyThrProSerGluIleGlyTyrHisHisGlySer**
**AlaAlaLysGlyGluIleAlaLysAlaIleAspPheAlaThrGlyLeuIleHisGly**
**LysThrLysLysThrGlnAlaGluLeuGluGlnLeuLeuArgGluLeuGluGlnVal**
**MetLysGlnArgTrpProArgTyrTyrGluGluIleCysGlyIleAlaLysGlyAla**
**GluArgGluValSerGluIleValMetLeuAsnThrArgThrGluPheAlaTyrGly**
**LeuValGluAlaArgAspGlyCysThrThrValTyrCysLysThrProAsnGlyAla**
**LeuGlnGlyGlnAsnTrpAspPhePheThrAlaThrLysGluAsnLeuIleGlnLeu**
**ThrIleCysGlnProGlyLeuProThrIleLysMetIleThrGluAlaGlyIleIle**
**GlyLysValGlyPheAsnSerAlaGlyValAlaValAsnTyrAsnAlaLeuHisLeu**
**HisGlyLeuArgProThrGlyLeuProSerHisLeuAlaLeuArgMetAlaLeuGlu**
**SerThrSerProSerGluAlaTyrGluLysIleValSerGlnGlyGlyMetAlaAla**
**SerAlaPheIleMetValGlyAsnAlaHisGluAlaTyrGlyLeuGluPheSerPro**
**IleSerLeuCysLysGlnValAlaAspThrAsnGlyArgIleValHisThrAsnHis**
**CysLeuLeuAsnHisGlyProSerAlaGlnGluLeuAsnProLeuProAspSerTrp**
**SerArgHisGlyArgMetGluHisLeuLeuSerGlyPheAspGlyThrLysGluAla**
**PheAlaLysLeuTrpGluAspGluAspAsnTyrProLeuSerIleCysArgAlaTyr**
**LysGluGlyLysSerArgGlySerThrLeuPheAsnIleValPheAspHisValGly**
**ArgLysAlaThrValArgLeuGlyArgProAsnAsnProAspGluThrPheValMet**
**ThrPheSerAsnLeuAspThrLysSerAlaIleGlnAlaAsnIle**

worin Ala für einen Alaninrest steht, Arg für einen Argininrest steht, Asn für einen Asparaginrest steht, Asp für einen Asparaginsäurerest steht, Cys für einen Cysteinrest steht, Gln für einen Glutaminrest steht, Glu für einen Glutaminsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht, Ser für einen Serinrest steht, Thr für einen Threoninrest steht. Trp für einen Tryptophanrest steht, Tyr für einen Tyrosinrest steht und Val für einen Valinrest steht.

**2.** DNA Verbindung nach Anspruch 1, worin der kodierende Strang die folgende isolierte DNA Sequenz enthält

ATG CTT CAC GTA ACT TGC CAA GGT ACC CCC TCC GAA GTA AGT
CCA TAC CCG AAT GTA TAT TTG CCC ATA TTT AAC GCC TAT ACT
TCC AGA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC
TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG
GGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA

worin A ein Desoxyadenylrest ist, G ein Desoxyguanylrest ist. C ein Desoxycytidylrest ist und T ein Thymidylrest ist.

3. Das ~2,1 kb Pvul-SphI Restriktionsfragment von Plasmid pOG04 (erhältlich von NRRL B-18171 und in Figur 3 gezeigt).

4. Rekombinanter DNA Vektor, der die isolieite DNA Sequenz nach Anspruch 1 enthält.

5. Rekombinanter DNA Vektor nach Anspruch 4, der pOW1061 (erhältlich wie in Beispiel 4 beschrieben und in Figur 8 gezeigt) oder pOW1062 ist (erhältlich wie in Beispiel 8 beschrieben und in Figur 9 gezeigt).

6. Rekombinanter DNA Vektor nach Anspruch 4, der ferner einen Promotor und eine Translationsaktivierungssequenz enthält, die zur Expressionssteuerung dieser Acyltransferaseaktivitätkodierenden DNA positioniert ist.

7. Rekombinanter DNA Expressionsvektor nach Anspruch 6, worin der Promotor und die Translationsaktivierungssequenz in E. coli, Aspergillus, Penicillium oder Cephalosporium funktionsfähig ist.

8. Rekombinanter DNA Expressionsvektor nach Anspruch 7, worin dieser Promotor der E. coli λpL Promotor ist.

9. Rekombinanter DNA Expressionsvektor nach Anspruch 8, worin dieser Promotor der Promotor des Penicillium chrysogenum IPNS Gens ist.

10. Rekombinanter DNA Expressionsvektor nach Anspruch 8, worin dieser Promotor und die Translationsaktivierungssequenz in Cephalosporium funktionsfähig sind.

11. Rekombinanter DNA Vektor nach Anspruch 10, worin dieser Promotor der Promotor des Cephalosporium acremonium IPNS Gens ist.

12. Verfahren zur Konstruktion einer rekombinanten Wirtszelle, die zur Expression einer Acyltransferaseaktivität fähig ist, gekennzeichnet durch
Transformation dieser Wirtszelle mit einem rekombinanten DNA Expressionsvektor, der enthält:

(a) Einen Promotor und eine Translationsaktivierungssequenz, die in dieser Wirtszelle funktionsfähig sind, und

(b) die DNA Sequenz nach Anspruch 1, die zur Expression von diesem Promotor und dieser Translationsaktivierungssequenz aus positioniert ist.

13. Verfahren zur Expression einer Acyltransferaseaktivität in einer rekombinanten Wirtszelle, gekennzeichnet durch Kultivierung dieser transformierten Wirtszelle nach Anspruch 12 unter Bedingungen, die zur Genexpression geeignet sind.

14. Verfahren nach Anspruch 13, worin diese rekombinante Wirtszelle E. coli, Penicillium oder Aspergillus ist.

15. Rekombinante Wirtszelle, die mit einem rekombinanten DNA Vektor nach Anspruch 4 transformiert ist. wobei diese transformierte Wirtszelle E. coli K12. Penicillium oder Aspergillus ist.

16. DNA Verbindung nach Anspruch 1, worin der kodierende Strang die folgende isolierte DNA Sequenz enthält

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT
CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

worin A ein Desoxyadenylrest ist, G ein Desoxyguanylrest ist. C ein Desoxycytidylrest ist und T ein Thymidylrest ist.

**Patentansprüche für folgenden Vertragsstaat : ES**

1. Verfahren zur Konstruktion einer rekombinanten Wirtszelle, die zur Expression einer Acyltransferaseaktivität fähig ist, gekennzeichnet durch
Transformation dieser Wirtszelle mit einem rekombinanten DNA Expressionsvektor, der enthält:

(a) Einen Promotor und eine Translationsaktivierungssequenz, die in dieser Wirtszelle funktionsfähig sind, und

(b) eine DNA Sequenz, die zur Expression von diesem Promotor und dieser Translationsaktivierungssequenz aus positioniert ist, wobei diese DNA Sequenz kodiert für

MetLeuHisValThrCysGlnGlyThrProSerGluIleGlyTyrHisHisGlySer
AlaAlaLysGlyGluIleAlaLysAlaIleAspPheAlaThrGlyLeuIleHisGly
LysThrLysLysThrGlnAlaGluLeuGluGlnLeuLeuArgGluLeuGluGlnVal
MetLysGlnArgTrpProArgTyrTyrGluGluIleCysGlyIleAlaLysGlyAla
GluArgGluValSerGluIleValMetLeuAsnThrArgThrGluPheAlaTyrGly
LeuValGluAlaArgAspGlyCysThrThrValTyrCysLysThrProAsnGlyAla
LeuGlnGlyGlnAsnTrpAspPhePheThrAlaThrLysGluAsnLeuIleGlnLeu
ThrIleCysGlnProGlyLeuProThrIleLysMetIleThrGluAlaGlyIleIle
GlyLysValGlyPheAsnSerAlaGlyValAlaValAsnTyrAsnAlaLeuHisLeu
HisGlyLeuArgProThrGlyLeuProSerHisLeuAlaLeuArgMetAlaLeuGlu
SerThrSerProSerGluAlaTyrGluLysIleValSerGlnGlyGlyMetAlaAla
SerAlaPheIleMetValGlyAsnAlaHisGluAlaTyrGlyLeuGluPheSerPro
IleSerLeuCysLysGlnValAlaAspThrAsnGlyArgIleValHisThrAsnHis
CysLeuLeuAsnHisGlyProSerAlaGlnGluLeuAsnProLeuProAspSerTrp
SerArgHisGlyArgMetGluHisLeuLeuSerGlyPheAspGlyThrLysGluAla
PheAlaLysLeuTrpGluAspGluAspAsnTyrProLeuSerIleCysArgAlaTyr
LysGluGlyLysSerArgGlySerThrLeuPheAsnIleValPheAspHisValGly
ArgLysAlaThrValArgLeuGlyArgProAsnAsnProAspGluThrPheValMet
ThrPheSerAsnLeuAspThrLysSerAlaIleGlnAlaAsnIle

worin Ala für einen Alaninrest steht, Arg für einen Argininrest steht, Asn für einen Asparaginrest steht, Asp für einen Asparaginsäurerest steht, Cys für einen Cysteinrest steht, Gln für einen Glutaminrest steht, Glu für einen Glutaminsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht. Ser für einen Serinrest steht, Thr für einen Threoninrest steht, Trp für einen Tryptophanrest steht, Tyr für einen Tyrosinrest steht und Val für einen Valinrest steht.

**2.** Verfahren zur Expression einer Acyltransferaseaktivität in einer rekombinanten Wirtszelle, gekennzeichnet durch Kultivierung dieser transformierten Wirtszelle nach Anspruch 12 unter Bedingungen, die zur Genexpression geeignet sind.

**3.** Verfahren nach Anspruch 2, worin diese rekombinante Wirtszelle E. coli, Penicillium oder Aspergillus ist.

**4.** Verfahren nach einem der Ansprüche 1 bis 3, worin der kodierende Strang die folgende isolierte DNA Sequenz enthält

ATG CTT CAC GTA ACT TGC CAA GGT ACC CCC TCC GAA GTA AGT
CCA TAC CCG AAT GTA TAT TTG CCC ATA TTT AAC GCC TAT ACT
TCC AGA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC
TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG
GGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA

worin A ein Desoxyadenylrest ist, G ein Desoxyguanylrest ist, C ein Desoxycytidylrest ist und T ein Thymidylrest ist.

5. Verfahren nach einem der Ansprüche 1 bis 3, worin der kodierende Strang die folgende isolierte DNA Sequenz enthält

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT
CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

worin A ein Desoxyadenylrest ist, G ein Desoxyguanylrest ist, C ein Desoxycytidylrest ist und T ein Thymidylrest ist.

## Revendications

**Revendications pour les Etats contractants suivants : AT, BE, CH, DE, DK, FR, GB, GR, IT, LI, NL, SE**

**1.** Composé d'ADN qui comprend une séquence d'ADN isolée codant une activité d'acyltransférase isopénicilline N:acyl CoA de Aspergillus Nidulans, ladite séquence d'ADN isolée codant :

MetLeuHisValThrCysGlnGlyThrProSerGluIleGlyTyrHisHisGlySer
AlaAlaLysGlyGluIleAlaLysAlaIleAspPheAlaThrGlyLeuIleHisGly
LysThrLysLysThrGlnAlaGluLeuGluGlnLeuLeuArgGluLeuGluGlnVal
MetLysGlnArgTrpProArgTyrTyrGluGluIleCysGlyIleAlaLysGlyAla
GluArgGluValSerGluIleValMetLeuAsnThrArgThrGluPheAlaTyrGly
LeuValGluAlaArgAspGlyCysThrThrValTyrCysLysThrProAsnGlyAla
LeuGlnGlyGlnAsnTrpAspPhePheThrAlaThrLysGluAsnLeuIleGlnLeu
ThrIleCysGlnProGlyLeuProThrIleLysMetIleThrGluAlaGlyIleIle
GlyLysValGlyPheAsnSerAlaGlyValAlaValAsnTyrAsnAlaLeuHisLeu
HisGlyLeuArgProThrGlyLeuProSerHisLeuAlaLeuArgMetAlaLeuGlu
SerThrSerProSerGluAlaTyrGluLysIleValSerGlnGlyGlyMetAlaAla
SerAlaPheIleMetValGlyAsnAlaHisGluAlaTyrGlyLeuGluPheSerPro
IleSerLeuCysLysGlnValAlaAspThrAsnGlyArgIleValHisThrAsnHis
CysLeuLeuAsnHisGlyProSerAlaGlnGluLeuAsnProLeuProAspSerTrp
SerArgHisGlyArgMetGluHisLeuLeuSerGlyPheAspGlyThrLysGluAla
PheAlaLysLeuTrpGluAspGluAspAsnTyrProLeuSerIleCysArgAlaTyr
LysGluGlyLysSerArgGlySerThrLeuPheAsnIleValPheAspHisValGly
ArgLysAlaThrValArgLeuGlyArgProAsnAsnProAspGluThrPheValMet
ThrPheSerAsnLeuAspThrLysSerAlaIleGlnAlaAsnIle

dans laquelle Ala représente un résidu d'alanine, Arg représente un résidu d'arginine, Asn représente un résidu d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'histidine, Ile représente un résidu d'isoleucine, Leu représente un résidu de leucine, Lys représente un résidu de lysine, Met représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine.

**2.** Composé d'ADN selon la revendication 1, dans lequel le brin de codage comprend la séquence d'ADN isolée :

ATG CTT CAC GTA ACT TGC CAA GGT ACC CCC TCC GAA GTA AGT
CCA TAC CCG AAT GTA TAT TTG CCC ATA TTT AAC GCC TAT ACT
TCC ACA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC
TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG

CGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA

dans laquelle A représente un résidu désoxyadényle, G représente un résidu désoxyguanyle, C représente un

résidu désoxycytidyle et T représente un résidu thymidyle.

**3.** Fragment de restriction Pvul-SphI ~ 2,1 kb du plasmide pOGO4 (que l'on obtient à partir de NRRL B-18171 et représenté en figure 3).

**4.** Vecteur d'ADN recombinant qui comprend la séquence d'ADN isolée selon la revendication 1.

**5.** Vecteur d'ADN recombinant selon la revendication 4, à savoir pOW1061 (que l'on obtient comme décrit à l'exemple 4 et représenté en figure 8) ou pOW1062 (que l'on obtient comme décrit à l'exemple 8 et représenté en figure 9).

**6.** Vecteur d'ADN recombinant selon la revendication 4, qui comprend en outre une séquence de promoteur et d'activation de traduction positionnée pour diriger l'expression dudit ADN codant l'activité d'acyltransférase.

**7.** Vecteur d'expression d'ADN recombinant selon la revendication 6, dans lequel ladite séquence de promoteur et d'activation de traduction fonctionne dans E. coli, Aspergillus, Penicillium ou Cephalosporium.

**8.** Vecteur d'expression d'ADN recombinant selon la revendication 7, dans lequel ledit promoteur est le promoteur λpl de E. coli.

**9.** Vecteur d'expression d'ADN recombinant selon la revendication 8, dans lequel ledit promoteur est le promoteur du gène IPNS de Penicillium chrysogenum.

**10.** Vecteur d'expression d'ADN recombinant selon la revendication 8, dans lequel ladite séquence de promoteur et d'activation de traduction fonctionne dans Cephalosporium.

**11.** Vecteur d'ADN recombinant selon la revendication 10, dans lequel ledit promoteur est le promoteur du gène IPNS de Cephalosporium acremonium.

**12.** Procédé pour construire une cellule hôte recombinante capable d'exprimer l'activité d'acyltransférase, ledit procédé comprenant:

la transformation de ladite cellule hôte avec un vecteur d'expression d'ADN recombinant qui comprend :

(a) une séquence de promoteur et d'activation de traduction qui fonctionne dans ladite cellule hôte, et
(b) ladite séquence d'ADN selon la revendication 1 positionnée pour l'expression à partir de ladite séquence de promoteur et d'activation de la traduction.

**13.** Procédé pour exprimer l'activité d'acyltransférase dans une cellule hôte recombinante, ledit procédé comprenant :

la culture de ladite cellule hôte transformée selon la revendication 12 dans des conditions appropriées pour l'expression génique.

**14.** Procédé selon la revendication 13, dans lequel ladite cellule hôte recombinante est E. coli, Penicillium ou Aspergillus.

**15.** Cellule hôte recombinante transformée avec un vecteur d'ADN recombinant selon la revendication 4, dans laquelle ladite cellule hôte transformée est E. coli K12, Penicillium ou Aspergillus.

**16.** Composé d'ADN selon la revendication 1, dans lequel le brin de codage comprend la séquence d'ADN isolée :

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT
CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

dans laquelle A représente un résidu désoxyadényle, G représente un résidu désoxyguanyle, C représente un résidu désoxycytidyle et T représente un résidu thymidyle.

**Revendications pour l'Etat contractant suivant : ES**

**1.** Procédé pour construire une cellule hôte recombinante capable d'exprimer l'activité d'acyltransférase, ledit procédé comprenant:
la transformation de ladite cellule hôte avec un vecteur d'expression d'ADN recombinant qui comprend :

(a) une séquence de promoteur et d'activation de traduction qui fonctionne dans ladite cellule hôte, et

(b) une séquence d'ADN positionnée pour l'expression à partir de ladite séquence de promoteur et d'activation de la traduction, ladite séquence d'ADN codant :

MetLeuHisValThrCysGlnGlyThrProSerGluIleGlyTyrHisHisGlySer
AlaAlaLysGlyGluIleAlaLysAlaIleAspPheAlaThrGlyLeuIleHisGly
LysThrLysLysThrGlnAlaGluLeuGluGlnLeuLeuArgGluLeuGluGlnVal
MetLysGlnArgTrpProArgTyrTyrGluGluIleCysGlyIleAlaLysGlyAla
GluArgGluValSerGluIleValMetLeuAsnThrArgThrGluPheAlaTyrGly
LeuValGluAlaArgAspGlyCysThrThrValTyrCysLysThrProAsnGlyAla
LeuGlnGlyGlnAsnTrpAspPhePheThrAlaThrLysGluAsnLeuIleGlnLeu
ThrIleCysGlnProGlyLeuProThrIleLysMetIleThrGluAlaGlyIleIle
GlyLysValGlyPheAsnSerAlaGlyValAlaValAsnTyrAsnAlaLeuHisLeu
HisGlyLeuArgProThrGlyLeuProSerHisLeuAlaLeuArgMetAlaLeuGlu
SerThrSerProSerGluAlaTyrGluLysIleValSerGlnGlyGlyMetAlaAla
SerAlaPheIleMetValGlyAsnAlaHisGluAlaTyrGlyLeuGluPheSerPro
IleSerLeuCysLysGlnValAlaAspThrAsnGlyArgIleValHisThrAsnHis
CysLeuLeuAsnHisGlyProSerAlaGlnGluLeuAsnProLeuProAspSerTrp
SerArgHisGlyArgMetGluHisLeuLeuSerGlyPheAspGlyThrLysGluAla
PheAlaLysLeuTrpGluAspGluAspAsnTyrProLeuSerIleCysArgAlaTyr
LysGluGlyLysSerArgGlySerThrLeuPheAsnIleValPheAspHisValGly
ArgLysAlaThrValArgLeuGlyArgProAsnAsnProAspGluThrPheValMet
ThrPheSerAsnLeuAspThrLysSerAlaIleGlnAlaAsnIle

dans laquelle Ala représente un résidu d'alanine, Arg représente un résidu d'arginine, Asn représente un résidu d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'histidine, Ile représente un résidu d'isoleucine, Leu représente un résidu de leucine, Lys représente un résidu de lysine, Met représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine.

**2.** Procédé pour exprimer l'activité d'acyltransférase dans une cellule hôte recombinante, ledit procédé comprenant : la culture de ladite cellule hôte transformée selon la revendication 1 dans des conditions appropriées pour l'expression génique.

**3.** Procédé selon la revendication 2, dans lequel ladite cellule hôte recombinante est E. coli, Penicillium ou Aspergillus.

**4.** Procédé selon l'une quelconque des revendications 1 à 3, dans lequel le brin de codage comprend la séquence d'ADN isolée :

ATG CTT CAC GTA ACT TGC CAA GGT ACC CCC TCC GAA GTA AGT
CCA TAC CCG AAT GTA TAT TTG CCC ATA TTT AAC GCC TAT ACT
TCÇ AÇA TCG GCT ATC ACC ATG GCT CTG CTG CCA AAG GCG AGA
TTG CGA AAG CCA TTG ACT TCG CAA CTG GCC TCA TTC ATG GCA
AAA CAA AAA AGA CAC AGG CGG AGC TTG AAC AGC TCC TCA GGG
AGT TGG AGC AGG TGA TGA AAC AGC GCT GGC CGA GAT ACT ATG
AGG AAA TCT GCG GTA TGT TTA CCT ACG TTC TTT ACT TGC TGA
ATC ACC CCG TTG ACG GAA TTT TCA GGA ATC GCA AAG GGT GCG
GAA CGC GAA GTA TCG GAG ATT GTC ATG CTC AAC ACT CGT ACG
GAA TTC GCG TAC GGG CTC GTA GAA GCC CGG GAC GGG TGC ACC
ACT GTT TAC TGC AAA ACC CCC AAT GGA GCG CTA CAG GGC CAG
AAC TGG GAC GTA GGT TAT TAG ATC CAC GGT CTG CTA TCT ATT
TTC CTT GCT AAC CGC CAT TCC GGC AGT TCT TCA CCG CAA CCA
AAG AAA ACT TGA TCC AGT TAA CAA TTT GTC AGC CGG GTC TAC
CCA CTA TCA AAA TGA TTA CAG AAG CTG GTA TCA TTG GCA AAG
TGG GTT TCA ACA GTG CTG GTG TCG CTG TCA ATT ACA ATG CAC

TAC ACC TAC ATG GCC TCC GTC CCA CTG GCC TCC CCT CGC ATC
TCG CGC TGC GCA TGG CCC TCG AAA GTA CAT CTC CGT CTG AGG
CGT ATG AAA AAA TCG TCT CGC AAG GGG GCA TGG CGG CTA GCG
CGT TCA TCA TGG TGG GCA ACG CAC ACG AGG CCT ACG GGC TAG
AGT TCT CGC CCA TCA GCT TGT GCA AGC AAG TTG CTG ACA CCA
ATG GGC GGA TAG TGC ATA CGA ACC ACT GCC TCC TCA ACC ATG
GGC CAT CGG CGC AAG AGC TTA ATC CCC TGC CGG ACT CGT GGA
GCC GCC ACG GGC GGA TGG AAC ATC TCC TCT CTG GTT TTG ACG
GCA CGA AGG AGG CAT TTG CGA AGT TGT GGG AGG ACG AAG ACA
ACT ACC CTC TCT CGA TCT GCC GGG CAT ATA AGG AAG GGA AAA
GTA GAG GCT CCA CTC TTT TCA ACA TCG TCT TCG ATC ATG TGG
GCC GGA AGG CAA CAG TGC GGC TGG GCC GGC CCA ATA ACC CTG
ATG AGA CCT TTG TCA TGA CCT TTA GCA ATC TGG ATA CCA AGT
CCG CGA TCC AAG CCA ACA TTT GA

dans laquelle A représente un résidu désoxyadényle, G représente un résidu désoxyguanyle, C représente un résidu désoxycytidyle et T représente un résidu thymidyle.

**5.** Procédé selon l'une quelconque des revendications 1 à 3, dans lequel le brin de codage comprend la séquence d'ADN isolée :

ATG CTT CAC GTA ACT TGC CAA GGT ACC CCC TCC GAA ATC
GGC TAT CAC CAT GGC TCT GCT GCC AAA GGC GAG ATT GCG
AAA GCC ATT GAC TTC GCA ACT GGC CTC ATT CAT GGC AAA
ACA AAA AAG ACA CAG GCG GAG CTT GAA CAG CTC CTC AGG
GAG TTG GAG CAG GTG ATG AAA CAG CGC TGG CCG AGA TAC
TAT GAG GAA ATC TGC GGT ATC GCA AAG GGT GCG GAA CGC
GAA GTA TCG GAG ATT GTC ATG CTC AAC ACT CGT ACG GAA
TTC GCG TAC GGG CTC GTA GAA GCC CGG GAC GGG TGC ACC
ACT GTT TAC TGC AAA ACC CCC AAT GGA GCG CTA CAG GGC
CAG AAC TGG GAC TTC TTC ACC GCA ACC AAA GAA AAC TTG
ATC CAG TTA ACA ATT TGT CAG CCG GGT CTA CCC ACT ATC
AAA ATG ATT ACA GAA GCT GGT ATC ATT GGC AAA GTG GGT
TTC AAC AGT GCT GGT GTC GCT GTC AAT TAC AAT GCA CTA
CAC CTA CAT GGC CTC CGT CCC ACT GGC CTC CCC TCG CAT

CTC GCG CTG CGC ATG GCC CTC GAA AGT ACA TCT CCG TCT
GAG GCG TAT GAA AAA ATC GTC TCG CAA GGG GGC ATG GCG
GCT AGC GCG TTC ATC ATG GTG GGC AAC GCA CAC GAG GCC
TAC GGG CTA GAG TTC TCG CCC ATC AGC TTG TGC AAG CAA
GTT GCT GAC ACC AAT GGG CGG ATA GTG CAT ACG AAC CAC
TGC CTC CTC AAC CAT GGG CCA TCG GCG CAA GAG CTT AAT
CCC CTG CCG GAC TCG TGG AGC CGC CAC GGG CGG ATG GAA
CAT CTC CTC TCT GGT TTT GAC GGC ACG AAG GAG GCA TTT
GCG AAG TTG TGG GAG GAC GAA GAC AAC TAC CCT CTC TCG
ATC TGC CGG GCA TAT AAG GAA GGG AAA AGT AGA GGC TCC
ACT CTT TTC AAC ATC GTC TTC GAT CAT GTG GGC CGG AAG
GCA ACA GTG CGG CTG GGC CGG CCC AAT AAC CCT GAT GAG
ACC TTT GTC ATG ACC TTT AGC AAT CTG GAT ACC AAG TCC
GCG ATC CAA GCC AAC ATT TGA

dans laquelle A représente un résidu désoxyadényle, G représente un résidu désoxyguanyle. C représente un résidu désoxycytidyle et T représente un résidu thymidyle.

# FIG.1
## Acyltransferase Reactions

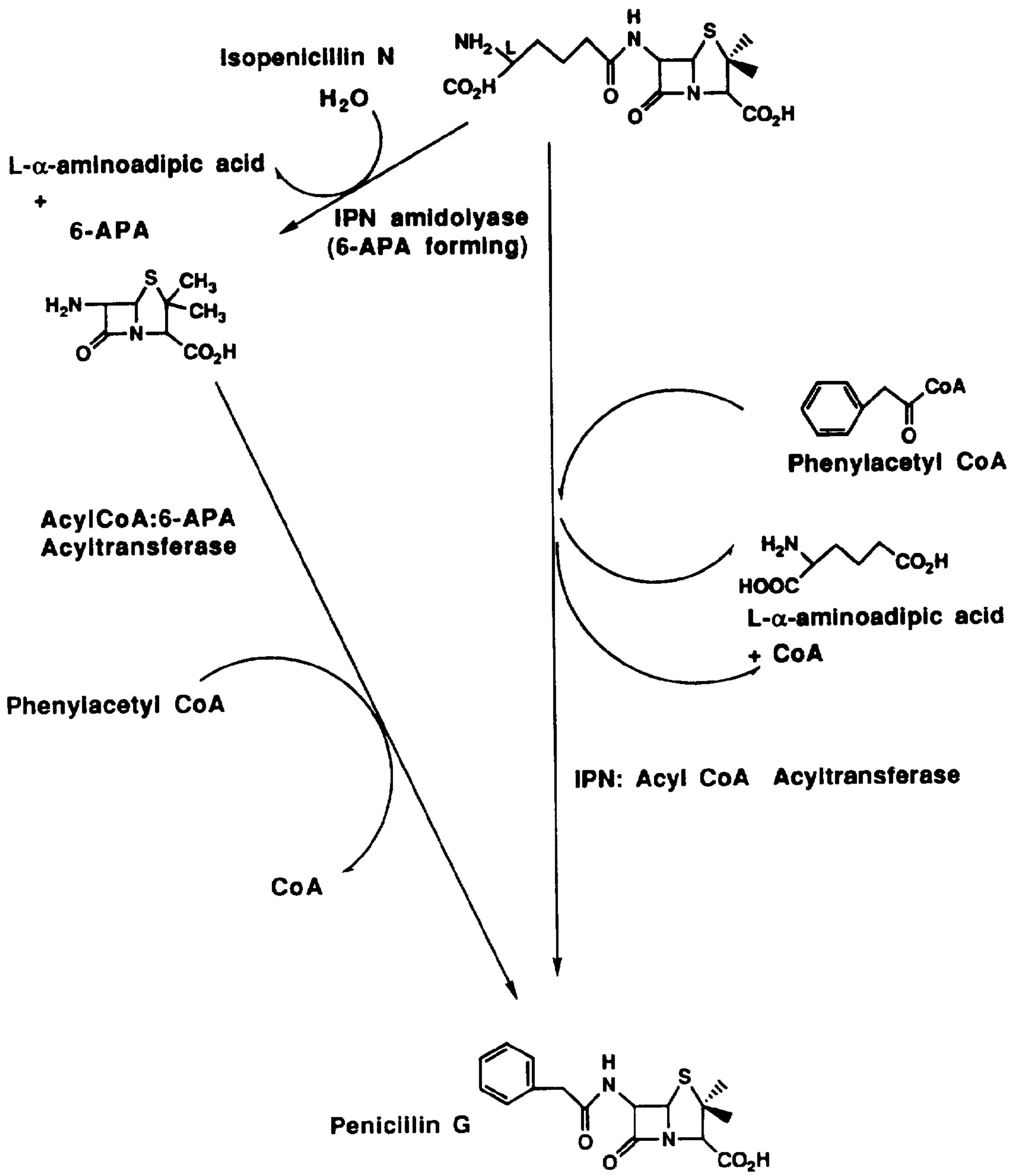

# FIG.2
# Beta-Lactam Biosynthesis

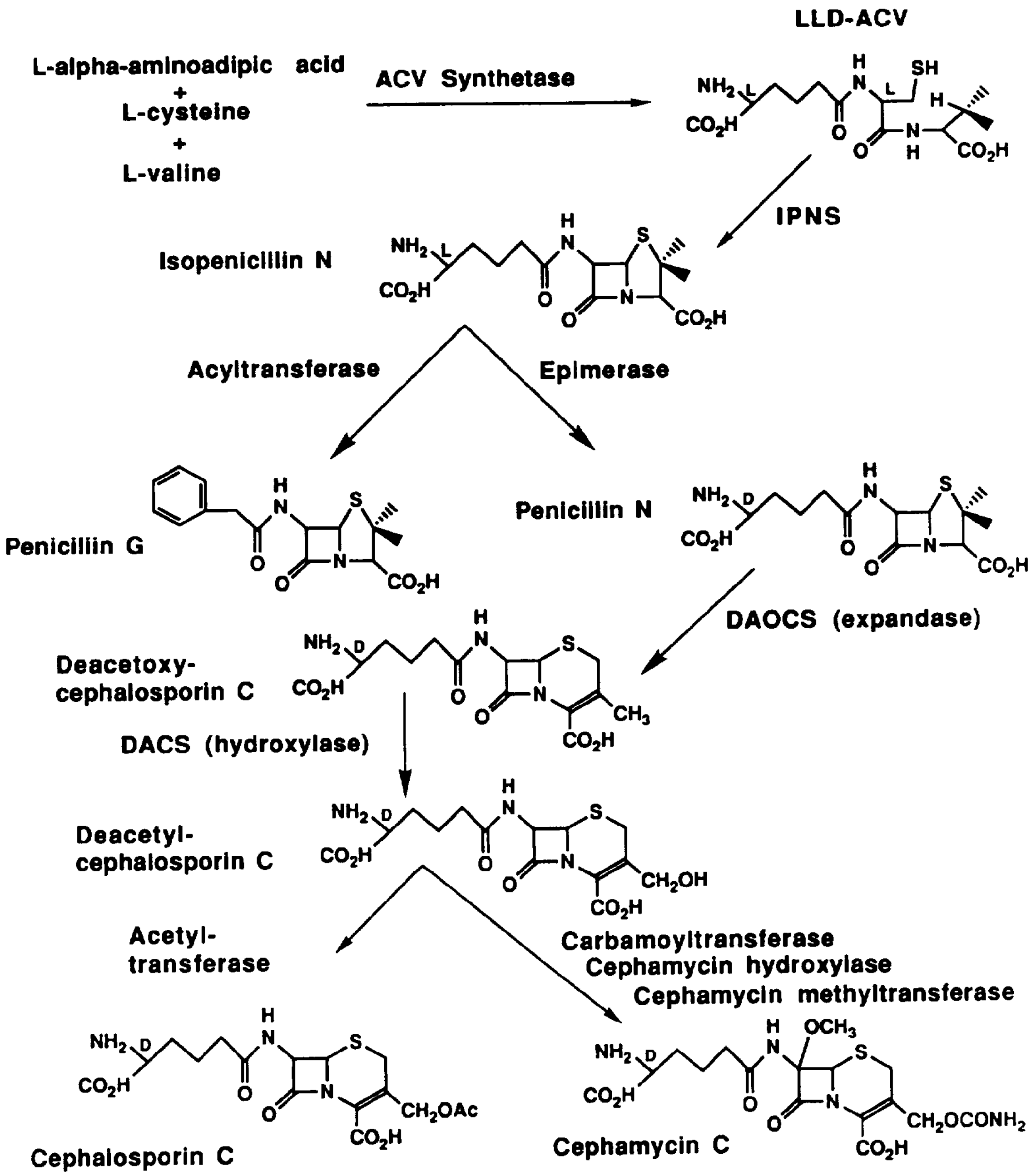


L-alpha-aminoadipic acid + L-cysteine + L-valine

LLD-ACV

Isopenicillin N

Penicillin G

Penicillin N

Deacetoxy-cephalosporin C

Deacetyl-cephalosporin C

Cephalosporin C

Cephamycin C

# FIG.3
# Restriction Site and Function Map of Plasmid pOGO4 (~7.2 kb)

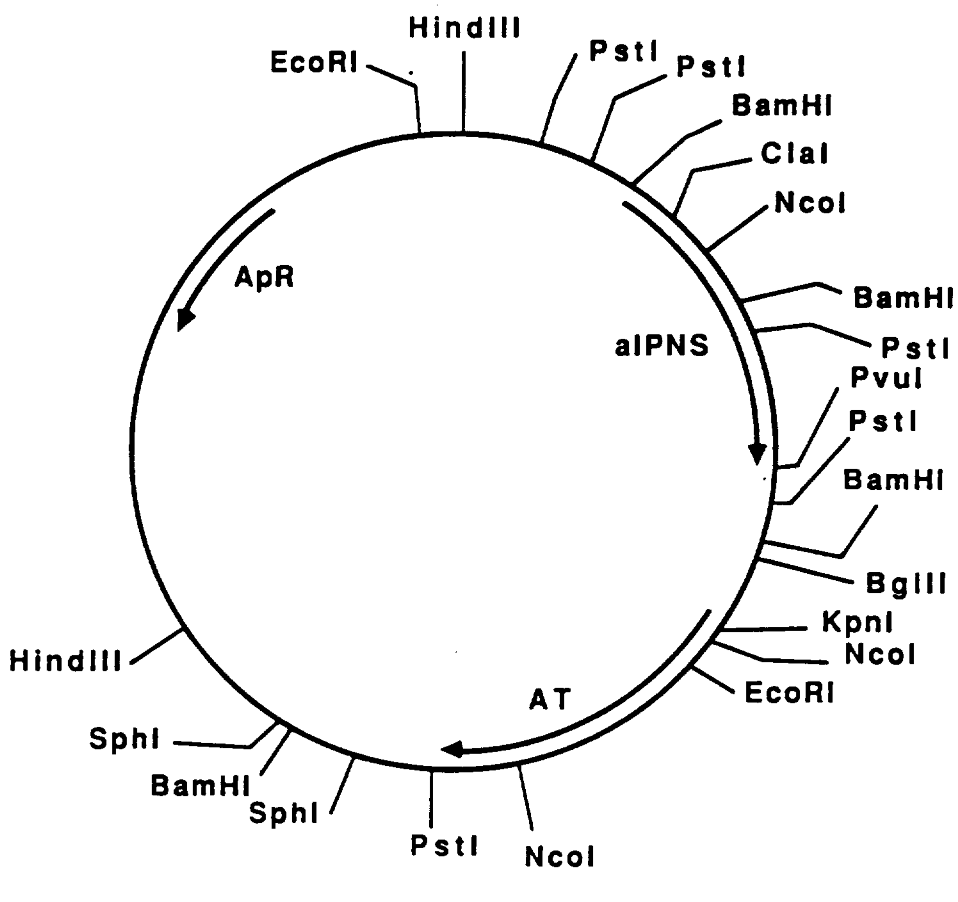

# FIG.4
## Restriction Site and Function Map of Plasmid pUT715 (~ 3.3 kb)

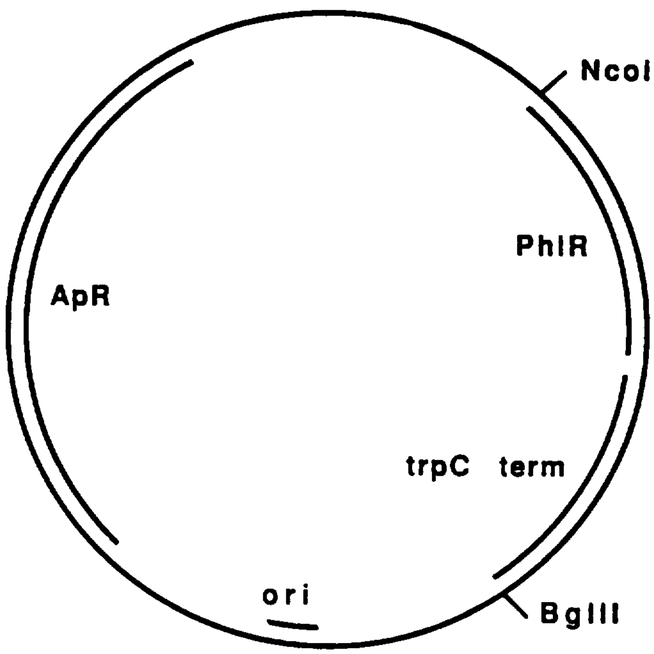

FIG.5
Restriction Site and Function Map of Plasmid pLC2 (~ 7.1 kb)

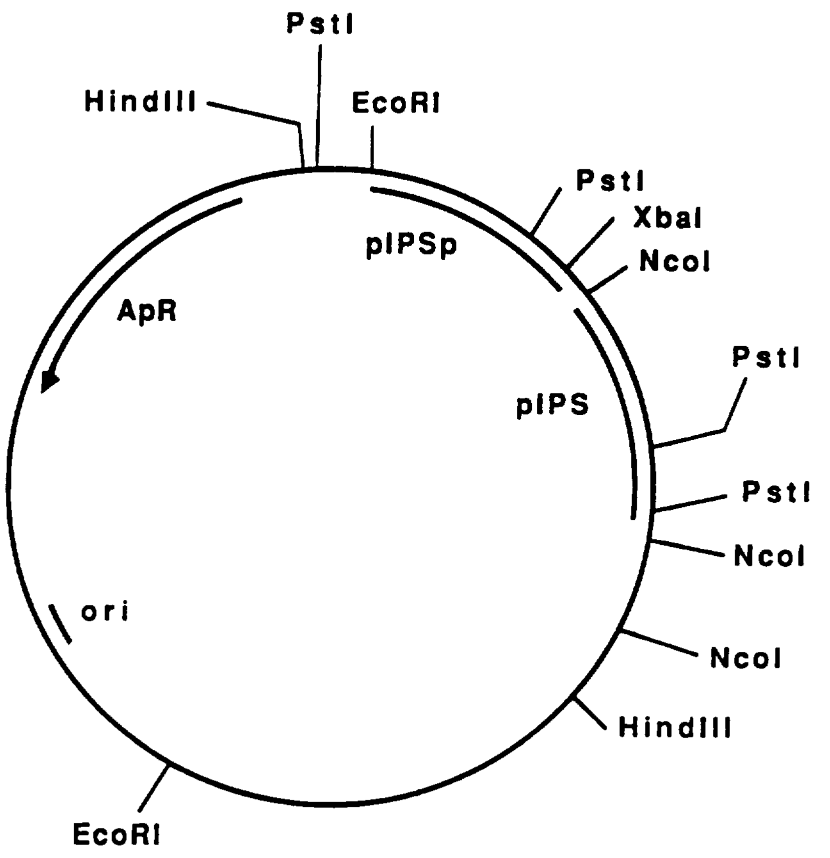

# FIG.6
# Restriction Site and Function Map of Plasmid pKC787 (~2.8 kb)

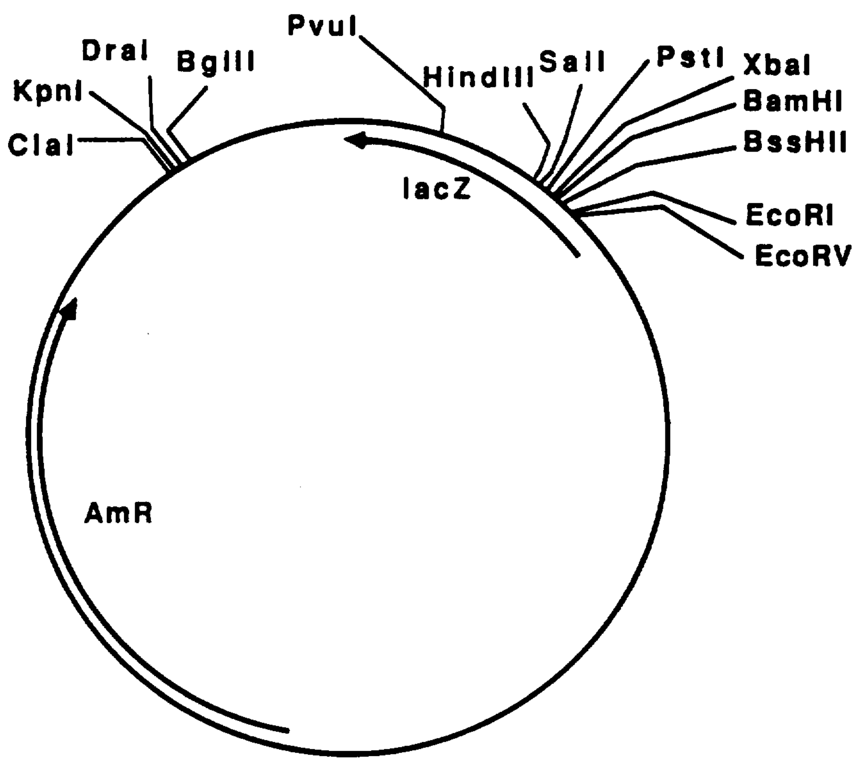

# FIG.7
## Restriction Site and Function Map of Plasmid pRH5 (~4.9 kb)

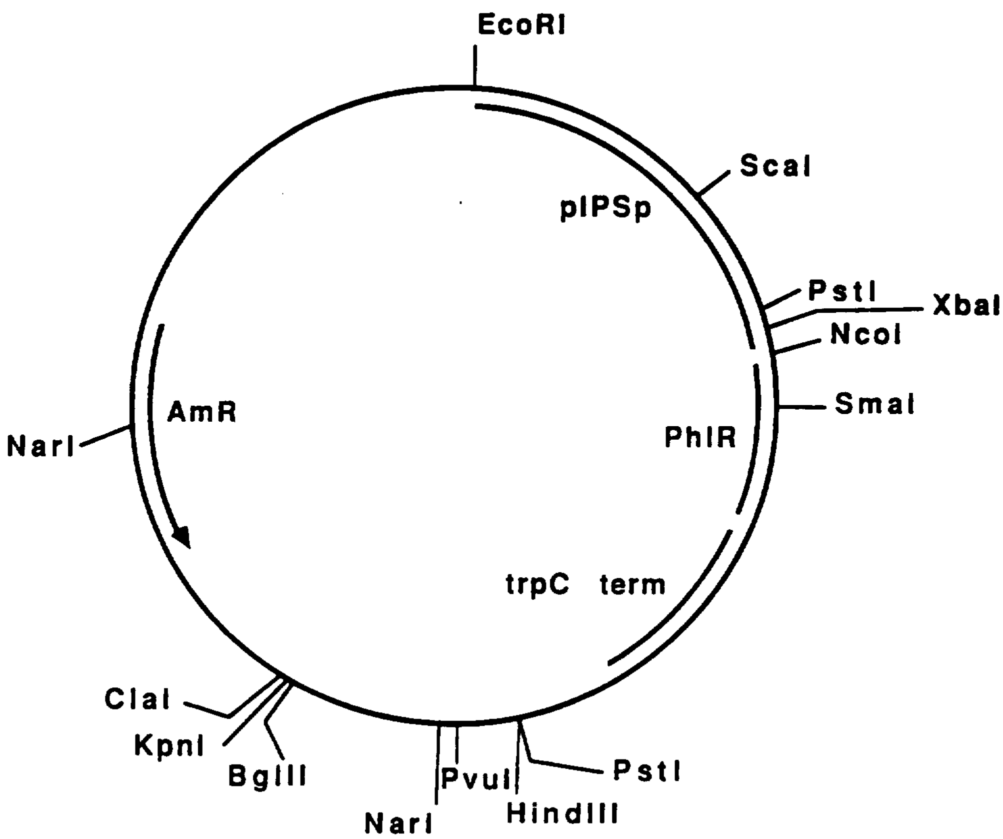

FIG.8
Restriction Site and Function Map of Plasmid pOW1061 (~6.8 kb)

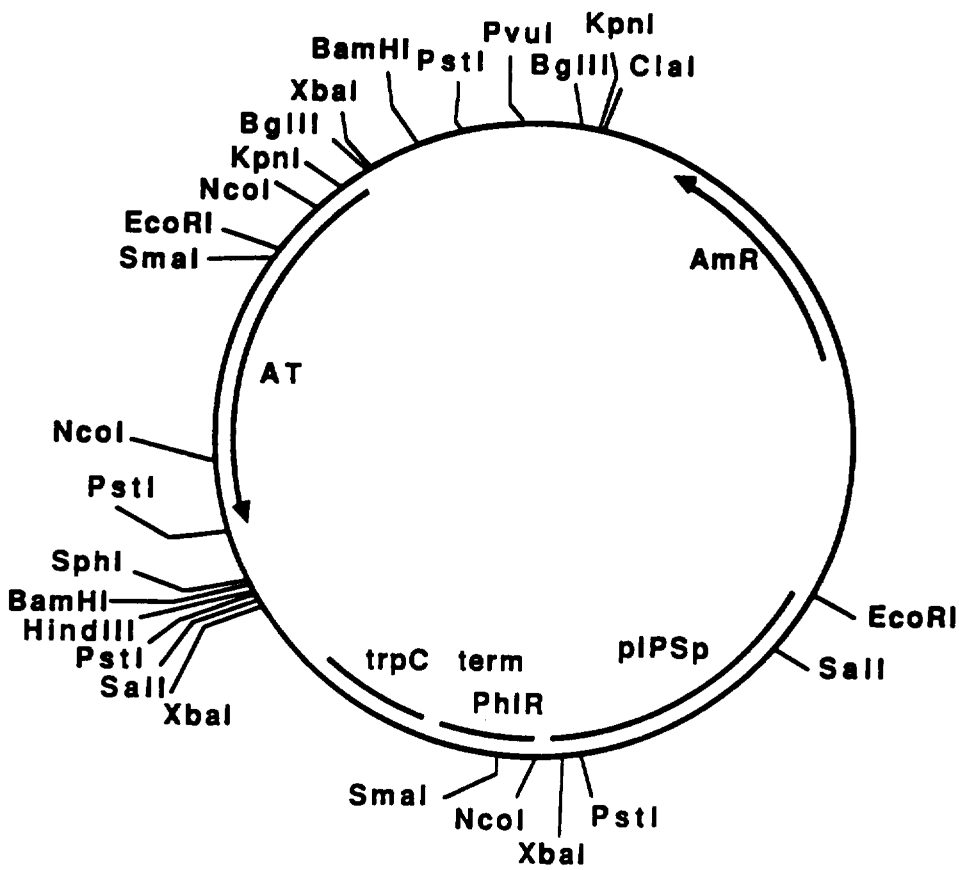

# FIG.9
## Restriction Site and Function Map of Plasmid pOW1062 (~8.0 kb)

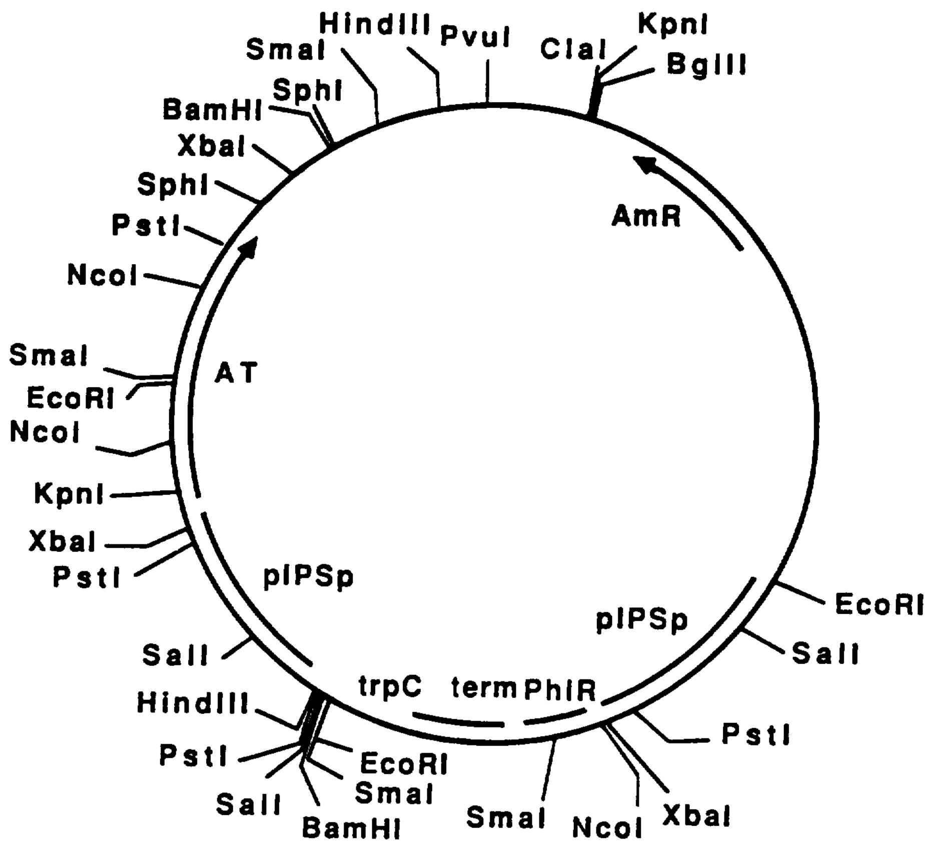

# FIG.10
# Restriction Site and Function Map of Plasmid pOW1063 (~5.7 kb)

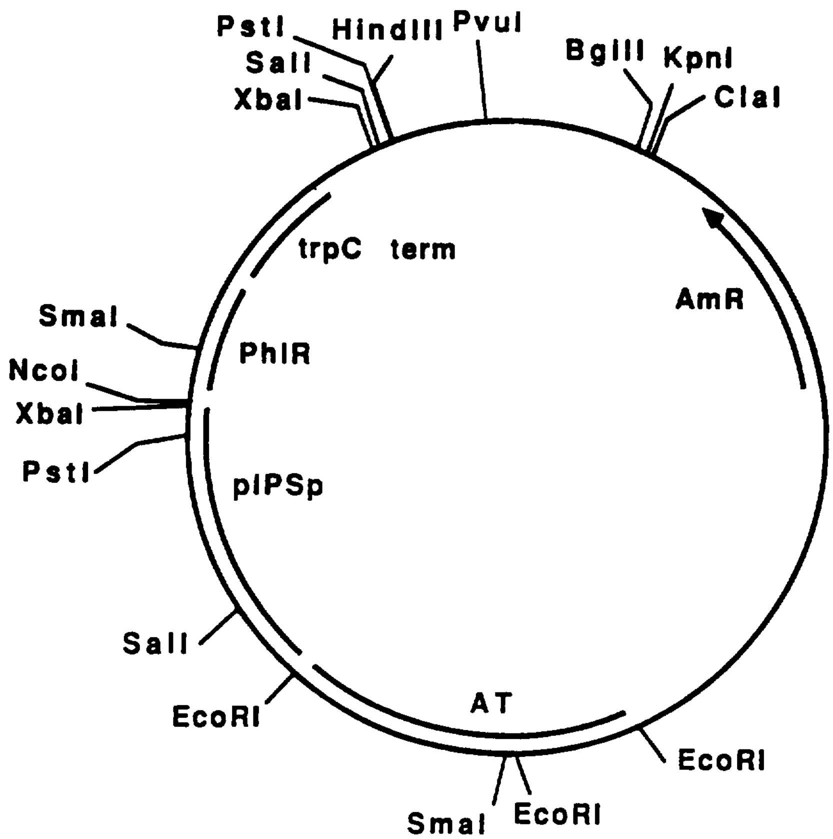

# FIG.11
## Restriction Site and Function Map of Plasmid pOW1064 (~5.5 kb)

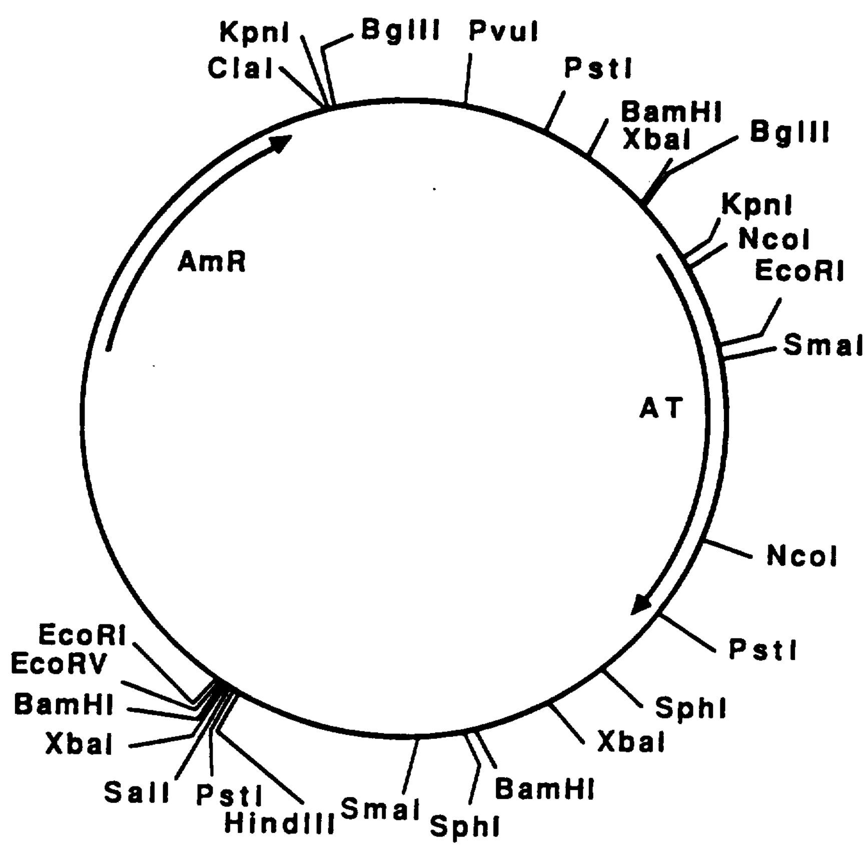

FIG.12

Restriction Site and Function Map of Plasmid pOW1065 (~6.8 kb)

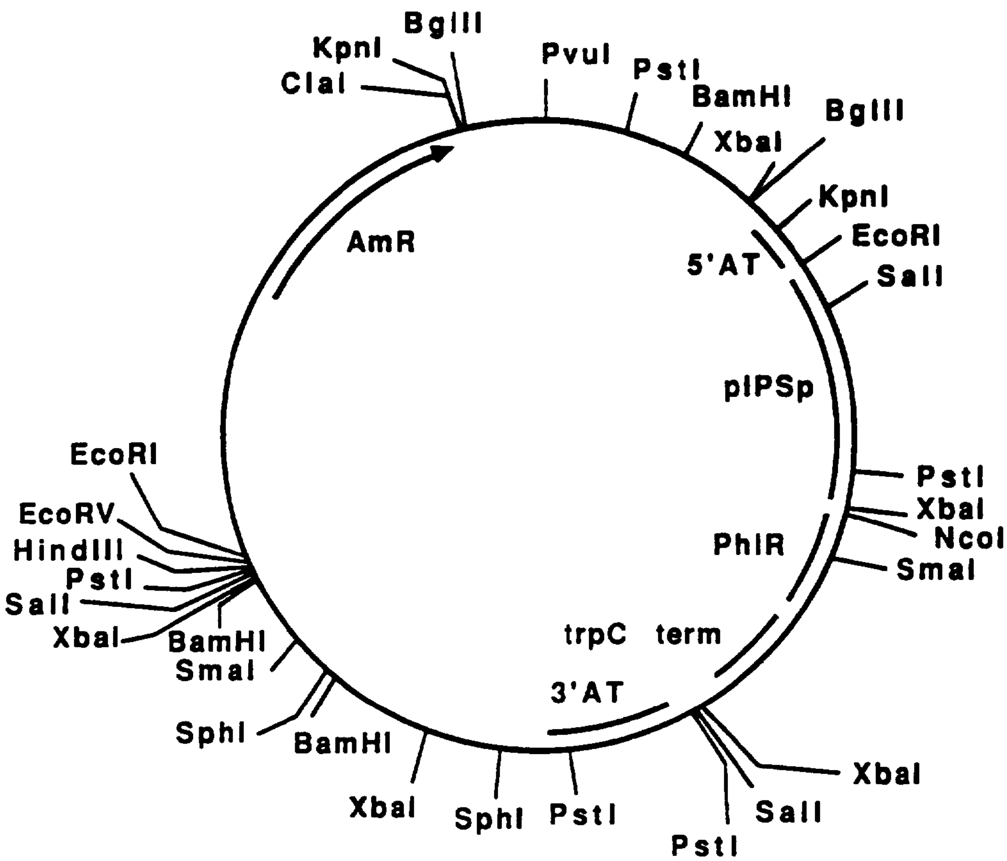

# FIG.13
# Restriction Site and Function Map of Plasmid pPS95 (~6.8 kb)